(19) Europäisches Patentamt
European Patent Office
Office européen des brevets

(11) EP 4 410 779 A1

(12) EUROPEAN PATENT APPLICATION
published in accordance with Art. 153(4) EPC

(43) Date of publication:
07.08.2024 Bulletin 2024/32

(21) Application number: 22875063.4

(22) Date of filing: 29.09.2022

(51) International Patent Classification (IPC):
*C07D 267/22 (2006.01)* *C07D 267/16 (2006.01)*
*C07D 498/22 (2006.01)* *A61K 31/33 (2006.01)*
*A61K 31/55 (2006.01)* *A61P 35/02 (2006.01)*

(52) Cooperative Patent Classification (CPC):
**A61K 31/33; A61K 31/55; A61P 35/02; C07D 267/16; C07D 267/22; C07D 498/22**

(86) International application number:
PCT/CN2022/122536

(87) International publication number:
WO 2023/051681 (06.04.2023 Gazette 2023/14)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: 30.09.2021 CN 202111161691

(71) Applicant: **Jiangsu Hansoh Pharmaceutical Group Co., Ltd.**
**Lianyungang, Jiangsu 222047 (CN)**

(72) Inventors:
- **WANG, Xiao**
  **Lianyungang, Jiangsu 222047 (CN)**
- **DING, Xiaohua**
  **Lianyungang, Jiangsu 222047 (CN)**
- **CHEN, Guohui**
  **Lianyungang, Jiangsu 222047 (CN)**
- **SUN, Yundong**
  **Lianyungang, Jiangsu 222047 (CN)**
- **WANG, Xiaolei**
  **Lianyungang, Jiangsu 222047 (CN)**

(74) Representative: **Cooley (UK) LLP**
**22 Bishopsgate**
**London EC2N 4BQ (GB)**

# (54) FOUR-MEMBERED FUSED RING COMPOUND AND PREPARATION METHOD AND USE THEREOF

(57) The present invention relates to four-membered fused cyclic compounds and preparation methods and uses thereof, and specifically relates to four-membered fused cyclic compounds, preparation methods thereof and pharmaceutical compositions containing a therapeutically effective amount of the compound, as well as uses thereof as tyrosine kinase inhibitors, especially in the preparation of drugs that inhibit the tyrosine kinase activity of a protein selected from the group consisting of ABL1 protein, ABL2-related proteins and chimeric protein BCR-ABL1.

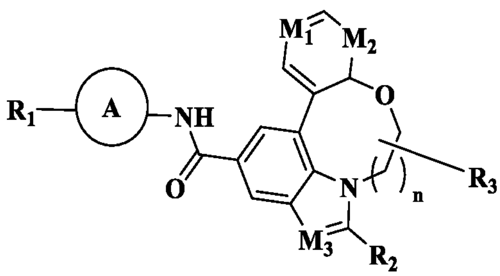

(I)

EP 4 410 779 A1

## Description

### FIELD OF THE INVENTION

The invention belongs to the field of medical biology, and specifically relates to four-membered fused cyclic compounds and preparation methods and uses thereof. It further relates to use of pharmaceutically acceptable salts thereof and pharmaceutical compositions thereof in the treatment of cancer and other related diseases.

### BACKGROUND OF THE INVENTION

The tyrosine kinase activity of ABL1 protein is usually strictly regulated, and the N-terminal capping region of the SH3 domain plays an important role here. One regulatory mechanism involves myristoylation of the N-terminal capping glycine-2 residue, which then interacts with the myristate binding site in the SH1 catalytic domain. A marker of chronic myelogenous leukemia (CML) is the Philadelphia chromosome (Ph), which is formed by the reciprocal translocation of the t(9,22) chromosomes in hematopoietic stem cells. This chromosome carries the BCR-ABL1 oncogene, which encodes a chimeric BCR-ABL1 protein lacking an N-terminal cap and having a constitutively active tyrosine kinase domain. Tyrosine kinase inhibitors (TKIs) for the BCR-ABL1 protein are standard treatments for CML patients, and imatinib, nilotinib, and dasatinib are approved for the treatment of newly diagnosed CML patients.

However, some patients develop drug-resistant clones in which mutations in the SHI domain can impair inhibitor binding. Although both nilotinib and dasatinib retain efficacy against many imatinib-resistant mutant forms of BCR-ABL1, mutations in which threonine 315 residue is substituted by isoleucine (T315I) are insensitive to all the three drugs and may cause CML patients to develop resistance to treatment. Therefore, inhibition of BCR-ABL1 mutations, such as T315I, still does not meet the medical needs. In addition to CML, BCR-ABL1 fusion protein is also causative in a certain proportion of acute lymphoblastic leukemia. Drugs targeting ABL kinase activity can also be used for this indication. Therefore, more BCR-ABL1 inhibitors are needed clinically.

### SUMMARY OF THE INVENTION

An objective of the present invention is to provide a compound represented by formula (I), stereoisomers thereof or pharmaceutically acceptable salts thereof:

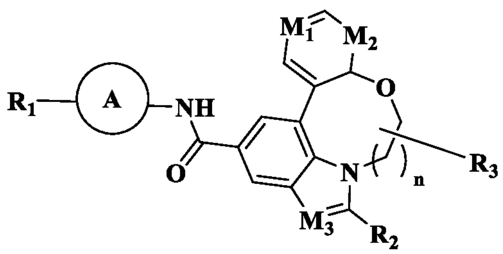

(I)

wherein:

- ring A is selected from $C_{3-6}$ cycloalkyl, 4-7-membered heterocyclyl, $C_{6-10}$ aryl or 5-7-membered heteroaryl;
- $R_1$ is selected from hydrogen, deuterium, halogen, amino, hydroxyl, cyano, $C_{1-6}$ alkyl, $C_{2-6}$ alkenyl, $C_{2-6}$ alkynyl, $C_{1-6}$ deuterated alkyl, $C_{1-6}$ haloalkyl, $C_{1-6}$ hydroxyalkyl, $C_{1-6}$ alkoxy, $C_{1-6}$ alkylthio or $C_{1-6}$ haloalkoxy;
- $M_1$ is selected from N or $CR_a$;
- $M_2$ is selected from $NR_a$ or $C(R_a)_2$;
- $M_3$ is selected from N or $CR_a$;
- $R_a$ is each independently selected from hydrogen, $C_{1-6}$ alkyl, $C_{1-6}$ deuterated alkyl, $C_{1-6}$ haloalkyl, $C_{1-6}$ hydroxyalkyl, $C_{1-6}$ alkoxy, $C_{1-6}$ alkylthio or $C_{1-6}$ haloalkoxy;
- $R_2$ or $R_3$ are each independently selected from hydrogen, $C_{1-6}$ alkyl, $C_{1-6}$ deuterated alkyl, $C_{1-6}$ haloalkyl, $C_{1-6}$ hydroxyalkyl, $C_{1-6}$ alkoxy, $C_{1-6}$ alkylthio, $C_{1-6}$ haloalkoxy, $C_{3-6}$ cycloalkyl, 4-7-membered heterocyclyl, $C_{6-10}$ aryl or 5-7-membered heteroaryl; and
- n is 0, 1 or 2.

In a preferred embodiment of the present invention, provided is the compound represented by formula (I),

stereoisomers thereof or pharmaceutically acceptable salts thereof, wherein:

ring A is $C_{6-10}$ aryl or 5-6-membered heteroaryl;
$R_1$ is selected from $C_{1-3}$ alkyl, $C_{1-3}$ deuterated alkyl, $C_{1-3}$ haloalkyl, $C_{1-3}$ hydroxyalkyl, $C_{1-3}$ alkoxy, $C_{13}$ alkylthio or $C_{1-3}$ haloalkoxy;
$M_1$ is selected from N or CH;
$M_2$ is selected from NH or $CH_2$;
$M_3$ is selected from N or CH; and
$R_2$ or $R_3$ are each independently selected from $C_{1-3}$ alkyl, $C_{1-3}$ deuterated alkyl, $C_{1-3}$ haloalkyl, $C_{1-3}$ hydroxyalkyl, $C_{1-3}$ alkoxy, $C_{1-3}$ alkylthio or $C_{1-3}$ haloalkoxy.

In a preferred embodiment of the present invention, the compound represented by formula (I) is further as described in general formula (II):

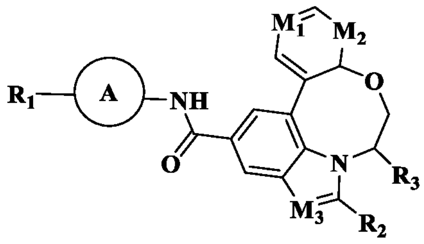

(II)

.

In a preferred embodiment of the present invention, the compound represented by formula (I) is further as described in general formula (II-a):

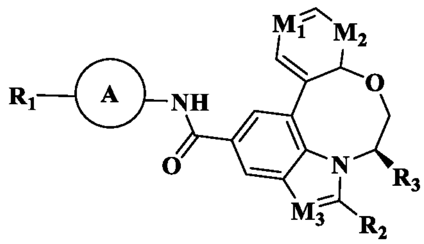

(II-a)

.

In a preferred embodiment of the present invention, the compound represented by formula (I) is further as described in general formula (II-b):

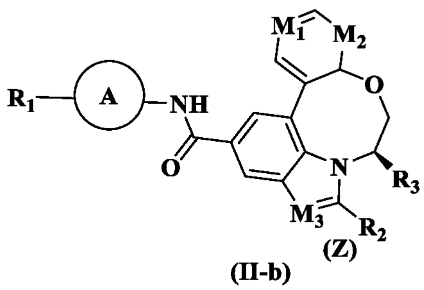

(Z)

(II-b)

.

In a more preferred embodiment of the invention, ring A is phenyl or pyridyl;

$R_1$ is selected from $C_{1-3}$ alkyl, $C_{1-3}$ haloalkyl, $C_{1-3}$ alkoxy or $C_{1-3}$ haloalkoxy;
$M_1$ is N;
$M_2$ is NH;

$M_3$ is N; and

$R_2$ or $R_3$ are each independently selected from hydrogen, $C_{1-3}$ alkyl or $C_{1-3}$ haloalkyl.

In a further preferred embodiment of the invention, $R_1$ is selected from $-CF_2$, $-CF_3$, $-CF_2Cl$, $-OCF_2$, $-OCF_3$ or $-OCF_2Cl$;

$R_2$ or $R_3$ is each independently selected from $-CH_3$, $-CF_2$, $-CF_3$ or $-CF_2Cl$.

In further preferred embodiments of the invention, the following specific compounds are included:

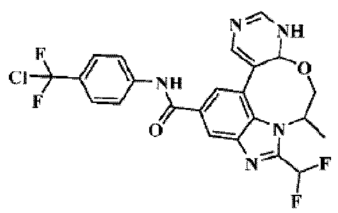
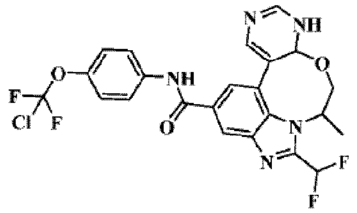
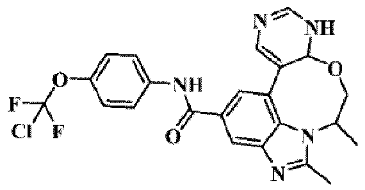
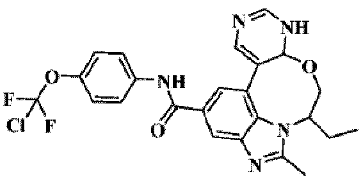
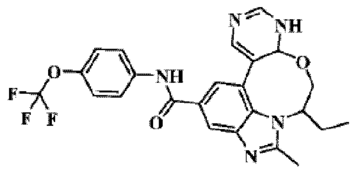
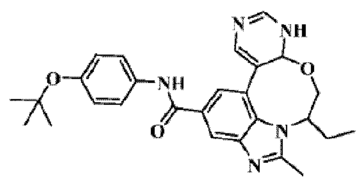
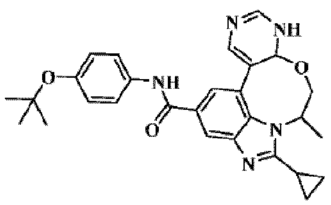
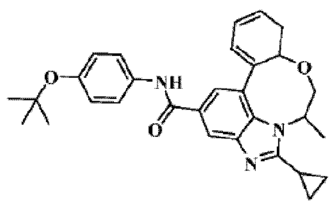
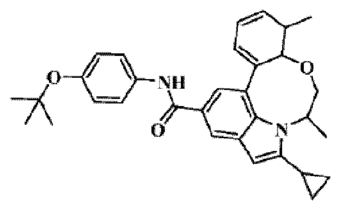
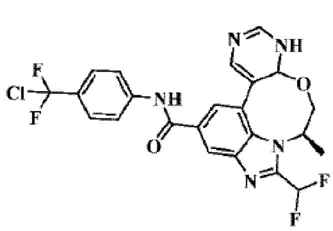
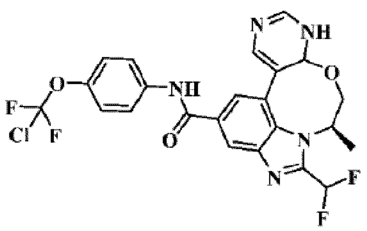
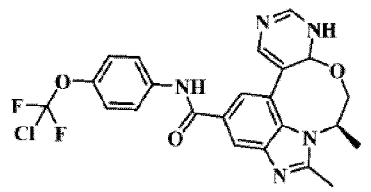
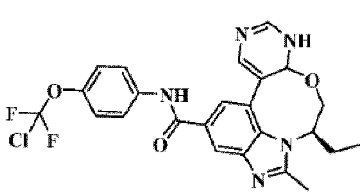
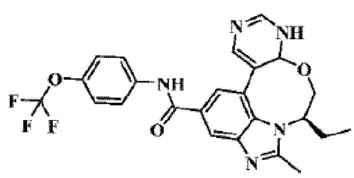
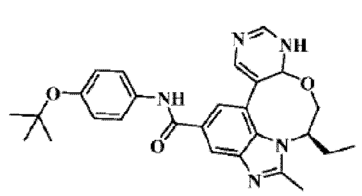
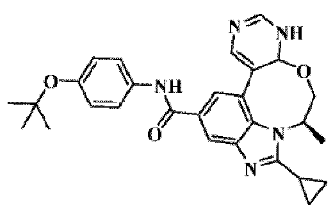
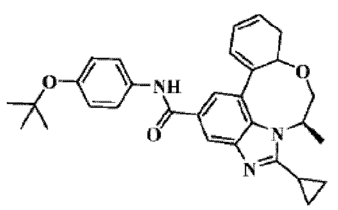
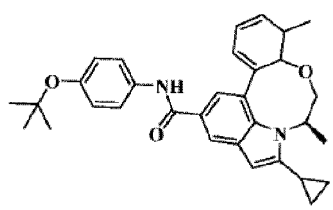
.

In a preferred embodiment of the present invention, the pharmaceutically acceptable salt is selected from sulfate, hydrochloride, ethyl sulfonate, methanesulfonate, p-toluenesulfonate, benzenesulfonate, hydroxyethyl sulfonate or 1,5-naphthalene disulfonate, more preferably p-toluenesulfonate.

In another aspect, the present invention provides a toluenesulfonate compound represented by formula (III):

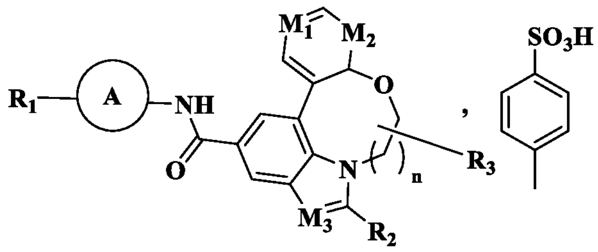

(III)

wherein:

- ring A is selected from $C_{3-6}$ cycloalkyl, 4-7-membered heterocyclyl, $C_{6-10}$ aryl or 5-7-membered heteroaryl;
- $R_1$ is selected from hydrogen, deuterium, halogen, amino, hydroxyl, cyano, $C_{1-6}$ alkyl, $C_{2-6}$ alkenyl, $C_{2-6}$ alkynyl, $C_{1-6}$ deuterated alkyl, $C_{1-6}$ haloalkyl, $C_{1-6}$ hydroxyalkyl, $C_{1-6}$ alkoxy, $C_{1-6}$ alkylthio or $C_{1-6}$ haloalkoxy;
- $M_1$ is selected from N or $CR_a$;
- $M_2$ is selected from $NR_a$ or $C(R_a)_2$;
- $M_3$ is selected from N or $CR_a$;
- $R_a$ is each independently selected from hydrogen, $C_{1-6}$ alkyl, $C_{1-6}$ deuterated alkyl, $C_{1-6}$ haloalkyl, $C_{1-6}$ hydroxyalkyl, $C_{1-6}$ alkoxy, $C_{1-6}$ alkylthio or $C_{1-6}$ haloalkoxy;
- $R_2$ or $R_3$ are each independently selected from hydrogen, $C_{1-6}$ alkyl, $C_{1-6}$ deuterated alkyl, $C_{1-6}$ haloalkyl, $C_{1-6}$ hydroxyalkyl, $C_{1-6}$ alkoxy, $C_{1-6}$ alkylthio, $C_{1-6}$ haloalkoxy, $C_{3-6}$ cycloalkyl, 4-7-membered heterocyclyl, $C_{6-10}$ aryl or 5-7-membered heteroaryl; and
- n is 0, 1 or 2.

In a preferred embodiment of the present invention, ring A is $C_{6-10}$ aryl or 5-6-membered heteroaryl;

- $R_1$ is selected from $C_{1-3}$ alkyl, $C_{1-3}$ deuterated alkyl, $C_{1-3}$ haloalkyl, $C_{1-3}$ hydroxyalkyl, $C_{1-3}$ alkoxy, $C_{13}$ alkylthio or $C_{1-3}$ haloalkoxy;
- $M_1$ is selected from N or CH;
- $M_2$ is selected from NH or $CH_2$;
- $M_3$ is selected from N or CH; and
- $R_2$ or $R_3$ are each independently selected from $C_{1-3}$ alkyl, $C_{1-3}$ deuterated alkyl, $C_{1-3}$ haloalkyl, $C_{1-3}$ hydroxyalkyl, $C_{1-3}$ alkoxy, $C_{1-3}$ alkylthio or $C_{1-3}$ haloalkoxy.

In a preferred embodiment of the present invention, the formula (III) is further as described in general formula (IV) or (IV-a):

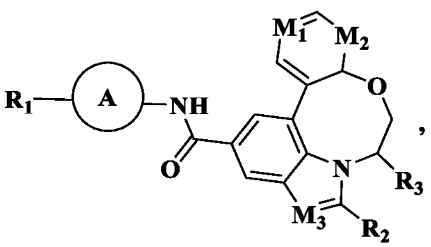

,

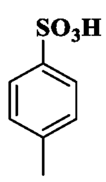

(IV)

or

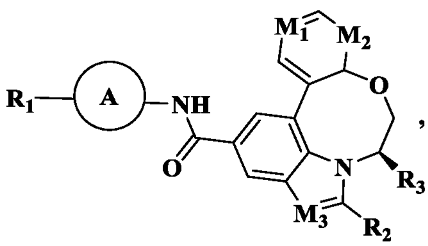

,

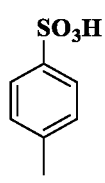

(IV-a)

.

In a preferred embodiment of the invention, ring A is phenyl or pyridyl;

- $R_1$ is selected from $C_{1-3}$ alkyl, $C_{1-3}$ haloalkyl, $C_{1-3}$ alkoxy or $C_{1-3}$ haloalkoxy;
- $M_1$ is N;
- $M_2$ is NH;
- $M_3$ is N; and

$R_2$ or $R_3$ are each independently selected from hydrogen, $C_{1-3}$ alkyl or $C_{1-3}$ haloalkyl.

In a preferred embodiment of the invention, $R_1$ is selected from $-CF_2$, $-CF_3$, $-CF_2Cl$, $-OCF_2$, - $OCF_3$ or $-OCF_2Cl$; and

$R_2$ or $R_3$ is each independently selected from $-CH_3$, $-CH_2CH_3$, $-CF_2$, $-CF_3$ or $-CF_2Cl$.

In a preferred embodiment of the present invention, the p-toluenesulfonate is selected from the following compounds:

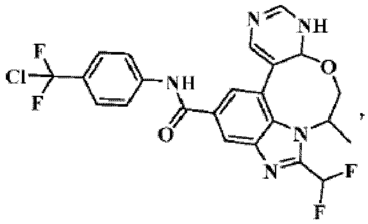 , 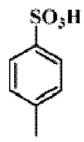

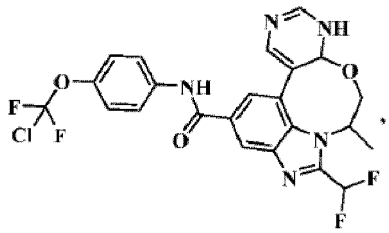 , 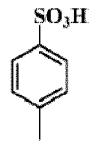

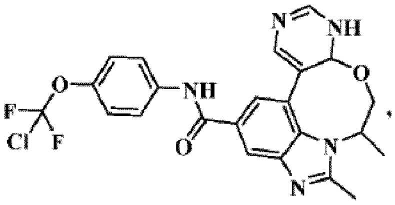 , 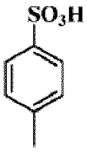

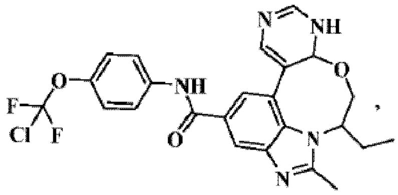 , 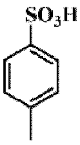

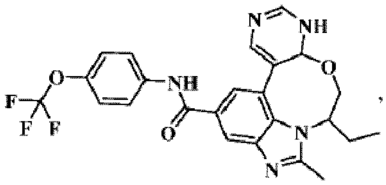 , 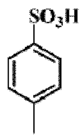

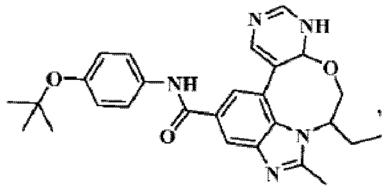 , 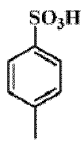

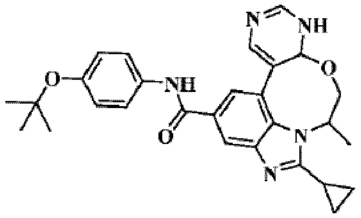 , 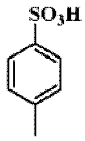

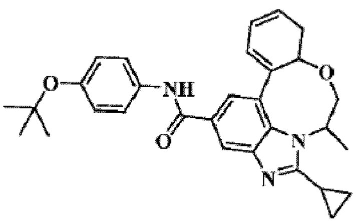 , 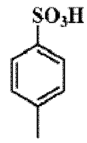

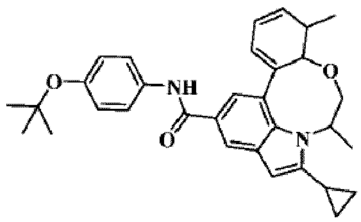 , 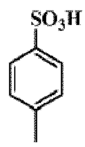

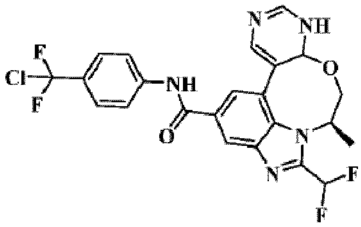 , 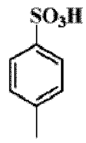

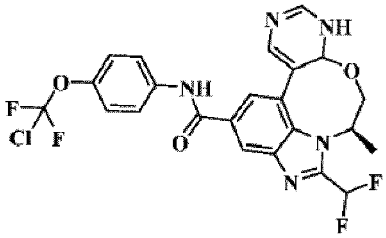 , 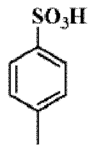 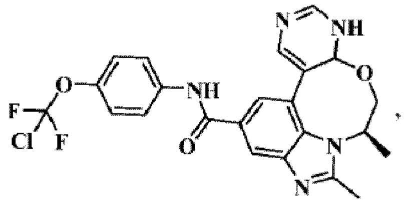 , 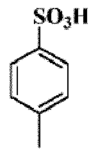

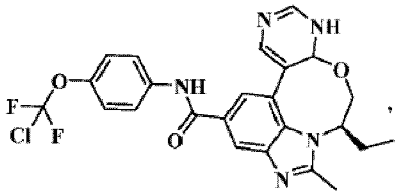 , 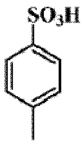 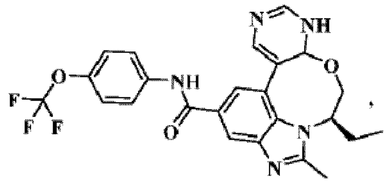 , 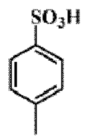

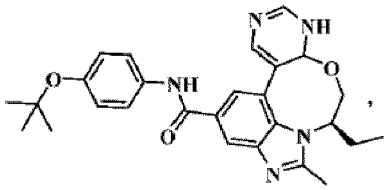 , 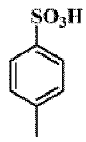 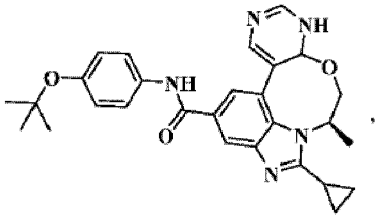 , 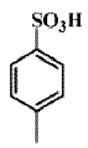

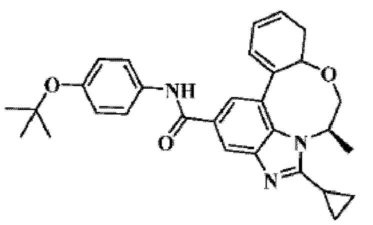 , 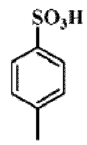 or 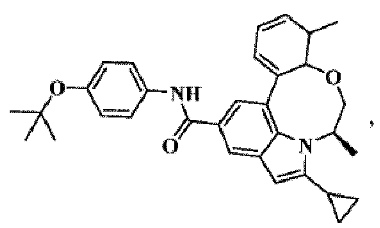 , 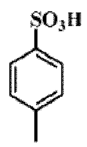 .

A further objective of the present invention is to provide a method for preparing a compound represented by formula (II), stereoisomers thereof or pharmaceutically acceptable salts thereof:

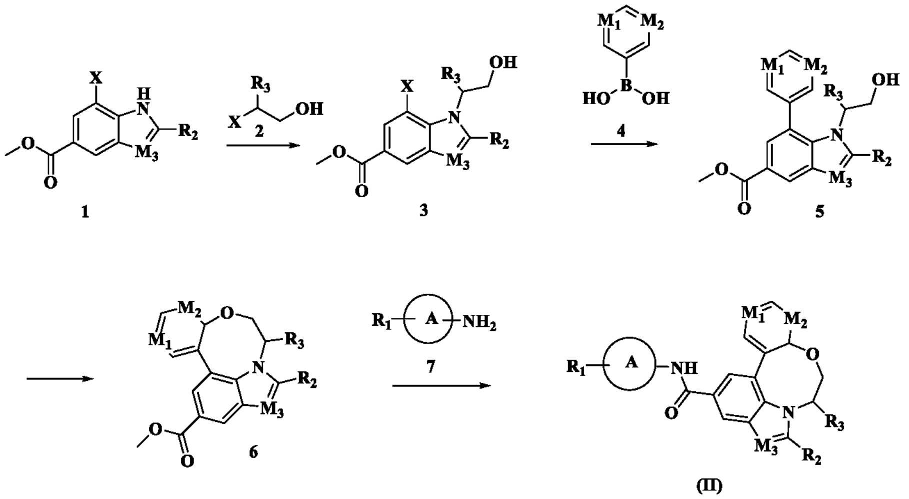


wherein compound 1 and compound 2 undergo substitution to prepare compound 3, compound 5 is prepared under the action of boric acid compound 4, compound 5 is prepared into cyclic compound 6 under acidic conditions, which is subjected to condensation with compound 7 for preparation of compound (II), a stereoisomer thereof or a pharmaceutically

acceptable salt thereof;

optionally, compound (II) reacts with an acid to prepare a salt compound;
X is selected from halogen;
the definitions of ring A, $R_1$-$R_3$ and $M_1$-$M_3$ are as defined in the general formula (II); and
the acid is preferably p-toluenesulfonic acid.

A further objective of the present invention is to provide a method for preparing a compound represented by formula (II), stereoisomers thereof or pharmaceutically acceptable salts thereof:

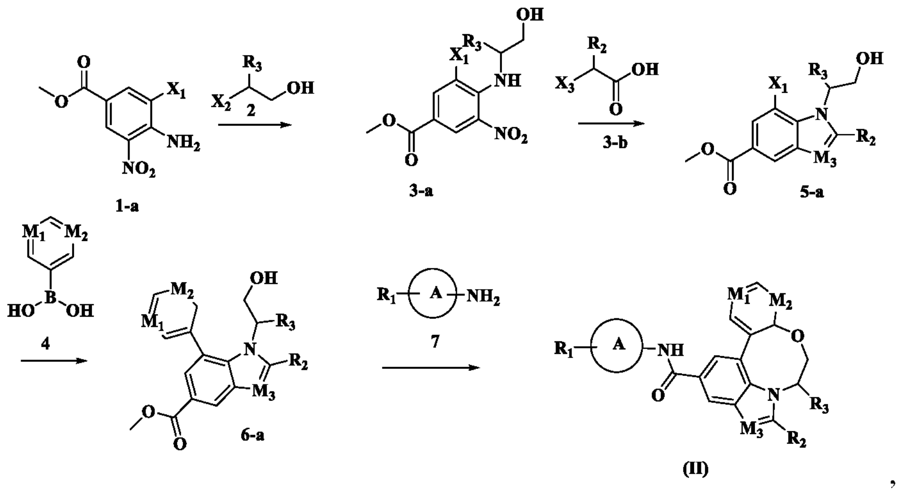
,

compound 1-a undergoes substitution with compound 2 to prepare compound 3-a, which reacts with 3-b to prepare compound 5-a, compound 6-a is prepared from compound 5-a under the action of boric acid compound 4, and compound 6-a and compound 7 undergo condensation to prepare compound (II);

optionally, compound (II) reacts with an acid to prepare a salt compound;
$X_1$, $X_2$ and $X_3$ are each independently selected from halogen;
the definitions of ring A, $R_1$-$R_3$ and $M_1$-$M_3$ are as defined in the general formula (II), preferably $M_3$ is N; and
the acid is preferably p-toluenesulfonic acid.

A further objective of the present application is to provide a pharmaceutical composition containing a therapeutically effective amount of the compound of formula (I), stereoisomers thereof or pharmaceutically acceptable salts thereof, and one or more pharmaceutically acceptable carriers or excipients.
The present invention further provides use of the compound of formula (I), stereoisomers thereof or pharmaceutically acceptable salts thereof, and the pharmaceutical composition in the preparation of a drug for inhibiting the tyrosine kinase enzyme activity of a protein selected from Abelson protein (ABL1), Abelson-related protein (ABL2) and chimeric protein BCR-ABL1.
The present invention further provides use of the compound of formula (I), stereoisomers thereof or pharmaceutically acceptable salts thereof, and the pharmaceutical composition thereof in the preparation of a drug for leukemia-related diseases.
In a preferred embodiment of the present invention, the leukemia is chronic myelogenous leukemia (CML), acute myeloid leukemia (AML) or acute lymphoblastic leukemia (ALL).
In a more preferred embodiment of the present invention, the CML is resistant to treatment with one or more of standard care treatments such as imatinib, nilotinib and dasatinib, and the AML is secondary AML, which develops following myelodysplastic syndrome (MDS) or myeloproliferative neoplasm (MPN).

## Detailed Description of the Invention

Unless stated to the contrary, the terms used in the specification and claims have the following meanings.
The term "alkyl" refers to a saturated aliphatic hydrocarbon group, which is a straight or branched chain group comprising 1 to 20 carbon atoms, preferably alkyl comprising 1 to 8 carbon atoms, more preferably alkyl of 1 to 6 carbon atoms, and most preferably alkyl of 1 to 3 carbon atoms. Non-limiting examples include methyl, ethyl, n-propyl, isopropyl,

n-butyl, isobutyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, n-heptyl, 2-methylhexyl, 3-methylhexyl, 4-methylhexyl, 5-methylhexyl, 2,3-dimethylpentyl, 2,4-dimethylpentyl, 2,2-dimethylpentyl, 3,3-dimethylpentyl, 2-ethylpentyl, 3-ethylpentyl, n-octyl, 2,3-dimethylhexyl, 2,4-dimethylhexyl, 2,5-dimethylhexyl, 2,2-dimethylhexyl, 3,3-dimethylhexyl, 4,4-dimethylhexyl, 2-ethylhexyl, 3-ethylhexyl, 4-ethylhexyl, 2-methyl-2-ethylpentyl, 2-methyl-3-ethylpentyl, n-nonyl, 2-methyl-2-ethylhexyl, 2-methyl-3-ethylhexyl, 2,2-diethylpentyl, n-decyl, 3,3-diethylhexyl, 2,2-diethylhexyl, and various branched isomers thereof, etc. More preferred are lower alkyl containing 1 to 6 carbon atoms, and non-limiting examples include methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, etc. The alkyl may be substituted or unsubstituted, and when it is substituted, the substituents may be substituted at any available linking point. The substituents are preferably one or more of groups independently selected from alkyl, alkenyl, alkynyl, alkoxy, alkylthio, alkylamino, halogen, mercapto, hydroxyl, nitro, cyano, cycloalkyl, heterocycloalkyl, aryl, heteroaryl, cycloalkoxy, heterocycloalkoxy, cycloalkylthio, heterocycloalkylthio, oxo, carboxyl or carboxylate. In the present invention, methyl, ethyl, isopropyl, tert-butyl, haloalkyl, deuterated alkyl, alkoxy-substituted alkyl and hydroxyl-substituted alkyl are preferred.

The term "cycloalkyl" refers to a saturated or partially unsaturated monocyclic or polycyclic cyclic hydrocarbon substituent. The cycloalkyl ring comprises 3 to 20 carbon atoms, preferably 3 to 12 carbon atoms, more preferably 3 to 8 carbon atoms, and most preferably 3 to 6 carbon atoms. Non-limiting examples of monocyclic cycloalkyl includes cyclopropyl, cyclobutyl, cyclopentyl, cyclopentenyl, cyclohexyl, cyclohexenyl, cyclohexadienyl, cycloheptyl, cycloheptatrienyl, cyclooctyl, etc.; and polycyclic cycloalkyl includes spiro, fused and bridged cycloalkyl, preferably cyclopropyl, cyclobutyl, cyclohexyl, cyclopentyl and cycloheptyl.

The term "spirocycloalkyl" refers to a 5 to 20-membered polycyclic group where the monocycles share a single carbon atom (called spiro atom), which may contain one or more double bonds, but none of the rings have a fully conjugated $\pi$ electron system. Preferably, it is 6 to 14-membered, more preferably 7 to 10-membered. According to the number of shared spiro atoms between the rings, the spirocycloalkyl is divided into mono-spirocycloalkyl, bi-spirocycloalkyl or poly-spirocycloalkyl, and is preferably mono-spirocycloalkyl and bi-spirocycloalkyl. More preferably, it is 3-membered/6-membered, 3-membered/5-membered, 4-membered/4-membered, 4-membered/5-membered, 4-membered/6-membered, 5-membered/5-membered or 5-membered/6-membered mono-spirocycloalkyl. Non-limiting examples of spirocycloalkyl include:

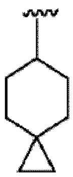 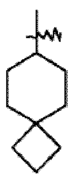 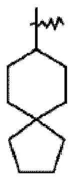 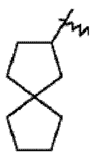 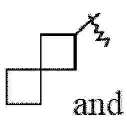 and 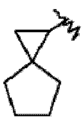 etc.;

Also included are spirocycloalkyl in which mono-spirocycloalkyl and heterocycloalkyl share a spiro atom, and non-limiting examples include:

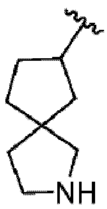 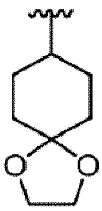 and 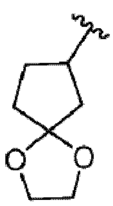 etc.

The term "fused cycloalkyl" refers to 5 to 20-membered all-carbon polycyclic groups where each of the rings in the system shares an adjacent pair of carbon atoms with the other rings in the system, wherein one or more rings may contain one or more double bonds, but none of the rings have a fully conjugated $\pi$ electron system. Preferably it is 6 to 14-membered, more preferably 7 to 10-membered. According to the number of constituent rings, it can be divided into bicyclic, tricyclic, tetracyclic or polycyclic fused cycloalkyl, preferably bicyclic or tricyclic, more preferably 5-membered/5-membered or 5-membered/6-membered bicyclic alkyl. Non-limiting examples of fused cycloalkyl include:

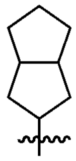 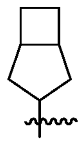 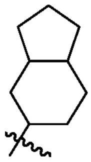 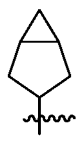 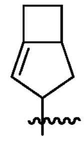 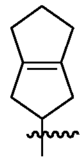 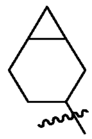 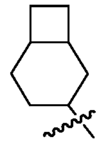 and 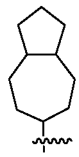, etc.

The term "bridged cycloalkyl" refers to a 5 to 20-membered all-carbon polycyclic group where, any two rings share two carbon atoms that are not directly connected, which may contain one or more double bonds, but none of the rings have a fully conjugated π electron system. Preferably, it is 6 to 14-membered, more preferably 7 to 10-membered. According to the number of constituent rings, it can be divided into bicyclic, tricyclic, tetracyclic or polycyclic bridged cycloalkyl, preferably bicyclic, tricyclic or tetracyclic, more preferably bicyclic or tricyclic. Non-limiting examples of bridged cycloalkyl include:

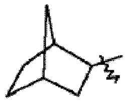 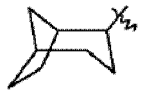 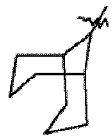 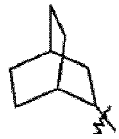 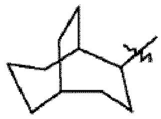

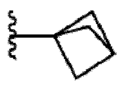 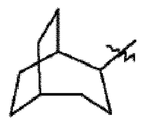 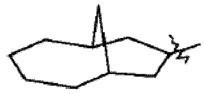 and 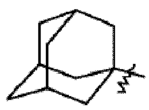.

The term "heterocyclyl" refers to a saturated or partially unsaturated monocyclic or polycyclic hydrocarbon substituent comprising 3 to 20 cyclic atoms, wherein one or more of the cyclic atoms are a heteroatom selected from nitrogen, oxygen, C(O) or $S(O)_m$ (where m is an integer of 0 to 2), but exclude the cyclic moieties of -O-O-, -O-S-, or -S-S-, and the remaining cyclic atoms are carbon. Preferably, it comprises 3 to 12 ring atoms, of which 1 to 4 are heteroatoms; more preferably, it comprises 3 to 8 ring atoms; most preferably, it comprises 3 to 8 ring atoms; further preferably, it is 3-8-membered heterocyclyl comprising 1-3 nitrogen atom, and optionally, it is substituted with 1-2 oxygen atoms, sulfur atoms, or oxo groups. It includes nitrogen-containing monocyclic heterocyclyl, nitrogen-containing spiroheterocyclyl, or nitrogen-containing fused heterocyclyl.

Non-limiting examples of monocyclic heterocyclyl include oxetanyl, azetidinyl, thietanyl, pyrrolidinyl, imidazolidinyl, tetrahydrofuranyl, tetrahydrothienyl, tetrahydropyranyl, dihydroimidazolyl, dihydrofuranyl, dihydropyrazolyl, dihydropyrrolyl, piperidinyl, piperazinyl, morpholinyl, thiomorpholinyl, homopiperazinyl, azepanyl, 1,4-diazepanyl, pyranyl or tetrahydrothiopyranyldioxide, etc.; preferably, oxetanyl, azetidinyl, thietanyl, tetrahydrofuranyl, tetrahydropyranyl, tetrahydrothiophenyl, tetrahydrothiopyranyl, tetrahydrothiopyranyldioxide, pyrrolidinyl, morpholinyl, piperidinyl, piperazinyl, hexahydropyrazinyl, hexahydropyrimidinyl, azepanyl, 1,4-diazepanyl and piperazinyl; and more preferably, piperidinyl, piperazinyl, pyrrolidinyl, morpholinyl, oxetanyl or azetidinyl. Polycyclic heterocyclyl includes spiro, fused and bridged heterocyclyl; the involved spiro, fused and bridged heterocyclyl are optionally connected to other groups through a single bond, or are further connected to other cycloalkyl, heterocyclyl, aryl and heteroaryl through any two or more atoms on the ring.

The term "spiroheterocyclyl" refers to a 5 to 20-membered polycyclic heterocyclic group where the monocyclic rings share one atom (called spiro atom), wherein one or more of the ring atoms are a heteroatom selected from nitrogen, oxygen or $S(O)_m$ (where m is an integer of 0 to 2), and the remaining ring atoms are carbon. It may contain one or more double bonds, but none of the rings has a fully conjugated π-electron system. Preferably, it is 5 to 12-membered, more preferably 7 to 10-membered. According to the number of shared spiro atoms between the rings, the spiroheterocyclyl is divided into mono-spiroheterocyclyl, bi-spiroheterocyclyl or poly-spiroheterocyclyl, and preferably mono-spiroheterocyclyl and bi-spiroheterocyclyl. More preferably, it is 3-membered/5-membered, 3-membered/6-membered, 4-membered/4-membered, 4-membered/5-membered, 4-membered/6-membered, 5-membered/5-membered or 5-membered/6-membered mono-spiroheterocyclyl. Non-limiting examples of spiroheterocyclyl include:

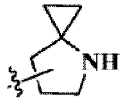 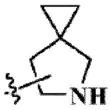 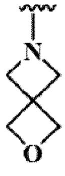 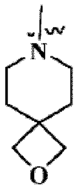 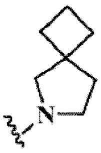 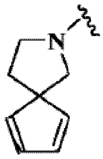 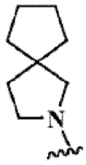 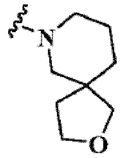

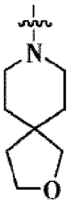 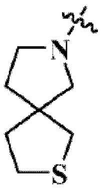 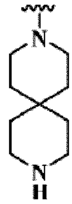 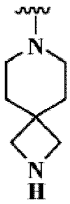 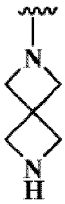 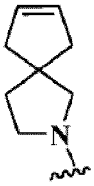 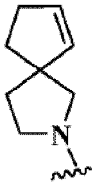 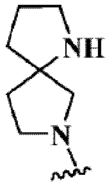 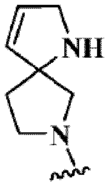 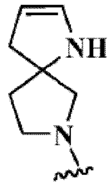 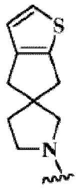

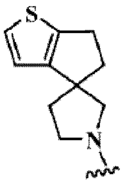 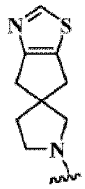 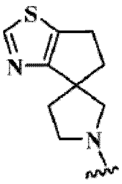 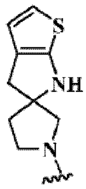 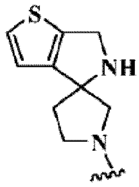 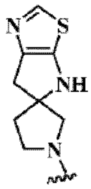 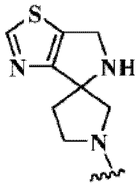 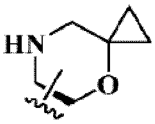 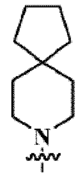

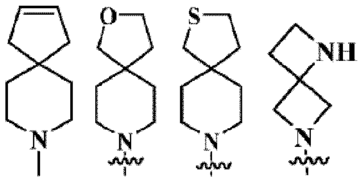

etc.

The term "fused heterocyclyl" refers to a 5 to 20-membered polycyclic heterocyclic group, where each of the rings in the system shares an adjacent pair of atoms with other rings in the system, and one or more rings may contain one or more double bonds, but none of the rings has a fully conjugated $\pi$ electron system, wherein one or more of the ring atoms are a heteroatom selected from nitrogen, oxygen, or $S(O)_m$ (where m is an integer of 0 to 2), and the remaining ring atoms are carbon. Preferably, it is 6 to 14-membered, more preferably 7 to 10-membered. According to the number of constituent rings, it can be divided into bicyclic, tricyclic, tetracyclic or polycyclic fused heterocyclyl, preferably bicyclic or tricyclic, more preferably 5-membered/5-membered or 5-membered/6-membered bicyclic fused heterocyclyl. Non-limiting examples of fused heterocyclyl include:

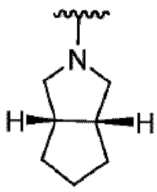 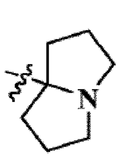 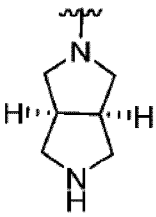 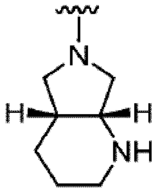 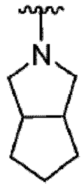 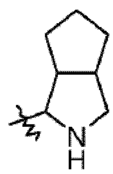 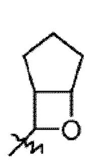

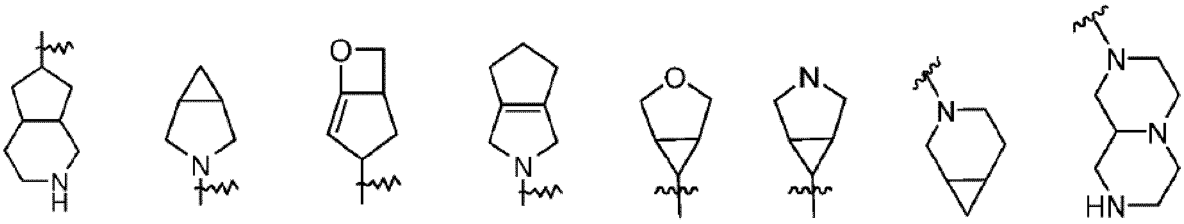

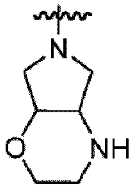 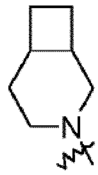 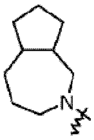 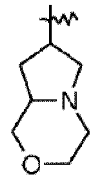 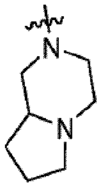 and 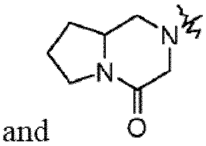

etc.

The term "bridged heterocyclyl" refers to a 5 to 14-membered polycyclic heterocyclic group, where any two rings share two atoms that are not directly connected, which may contain one or more double bonds, but none of the rings has a fully conjugated π electron system, wherein one or more of the ring atoms are a heteroatom selected from nitrogen, oxygen, or $S(O)_m$ (where m is an integer of 0 to 2), and the remaining ring atoms are carbon. Preferably, it is 6 to 14-membered, more preferably 6 to 10-membered. According to the number of constituent rings, it can be divided into bicyclic, tricyclic, tetracyclic or polycyclic bridged heterocyclyl, preferably bicyclic, tricyclic or tetracyclic, more preferably bicyclic or tricyclic. Non-limiting examples of bridged heterocyclyl include:

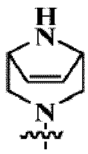 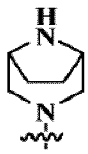 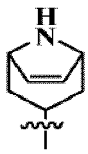 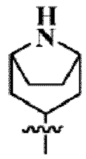 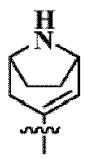 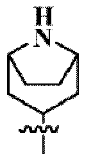 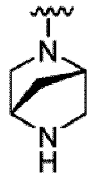 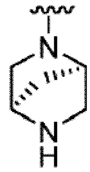 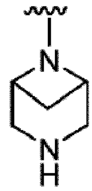 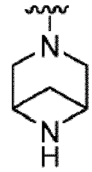

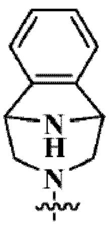 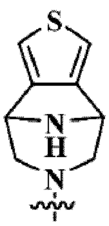 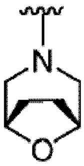 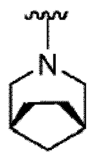 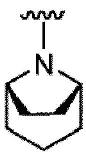 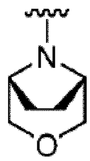 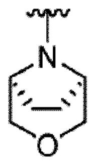 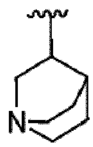 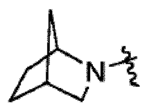

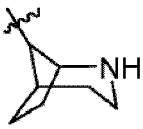 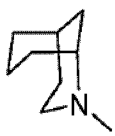 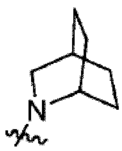 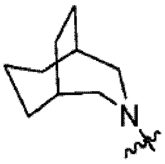 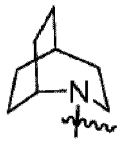 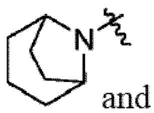 and 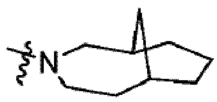

etc.

The heterocyclyl ring may be fused to an aryl, heteroaryl or cycloalkyl ring, wherein the ring connected to the parent structure is heterocyclyl, non-limiting examples of which include:

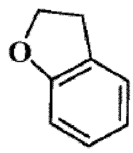 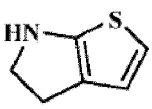 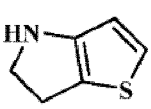 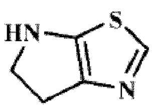 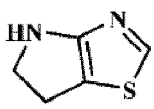 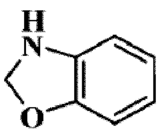 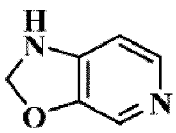

and

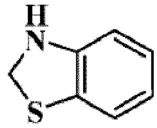

etc.

The heterocyclyl may be optionally substituted or unsubstituted. When it is substituted, the substituent is preferably one or more of groups independently selected from alkyl, alkenyl, alkynyl, alkoxy, alkylthio, alkylamino, halogen, mercapto, hydroxyl, nitro, cyano, cycloalkyl, heterocycloalkyl, aryl, heteroaryl, cycloalkoxy, heterocycloalkoxy, cycloalkylthio, heterocycloalkylthio, oxo, carboxyl or carboxylate.

The term "aryl" refers to a 6 to 14-membered, preferably 6 to 10-membered all-carbon monocyclic or fused polycyclic (i.e., rings sharing adjacent pairs of carbon atoms) group having a conjugated $\pi$ electron system, such as phenyl and naphthyl. More preferred, it is phenyl. The aryl ring can be fused onto a heteroaryl, heterocyclyl or cycloalkyl ring, including benzo-3-8-membered cycloalkyl, benzo-3-8-membered heteroalkyl, preferably benzo-3-6-membered cycloalkyl, benzo-3-6-membered heteroalkyl, wherein the heterocyclyl is a heterocyclic group comprising 1-3 nitrogen atoms, oxygen atoms, and sulfur atoms; or it also comprises a three-membered nitrogen-containing fused ring containing a benzene ring, wherein the ring connected to the parent structure is an aryl ring.

The aryl may be substituted or unsubstituted. When it is substituted, the substituent is preferably one or more of groups independently selected from alkyl, alkenyl, alkynyl, alkoxy, alkylthio, alkylamino, halogen, mercapto, hydroxyl, nitro, cyano, cycloalkyl, heterocycloalkyl, aryl, heteroaryl, cycloalkoxy, heterocycloalkoxy, cycloalkylthio, heterocycloalkylthio, carboxyl or carboxylate.

The term "heteroaryl" refers to a heteroaromatic system comprising 1 to 4 heteroatoms and 5 to 14 ring atoms, wherein the heteroatom is selected from oxygen, sulfur and nitrogen. The heteroaryl is preferably 5 to 10-membered, more preferably 5-membered or 6-membered, such as imidazolyl, furyl, thienyl, thiazolyl, pyrazolyl, oxazolyl, pyrrolyl, triazolyl, tetrazolyl, pyridyl, pyrimidinyl, thiadiazolyl, pyrazinyl, etc., preferably triazolyl, thienyl, imidazolyl, pyrazolyl, oxazolyl, pyrimidinyl or thiazolyl; more preferably, pyrazolyl and oxazolyl. The heteroaryl ring may be fused to an aryl, heterocyclyl or cycloalkyl ring, wherein the ring connected to the parent structure is heteroaryl ring, non-limiting examples of which include:

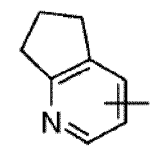 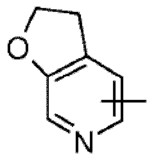 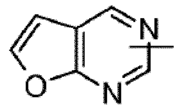 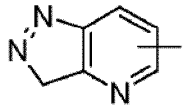 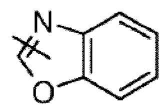 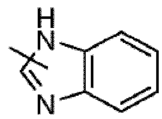

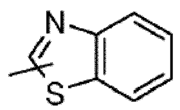 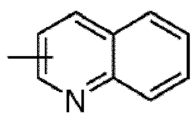 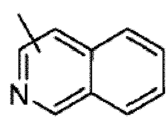 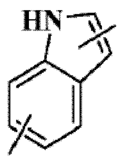 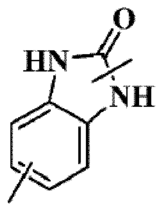 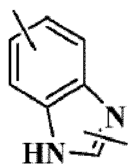 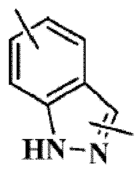

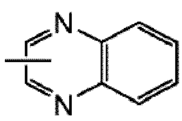 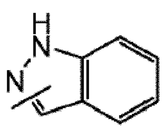 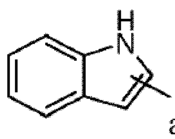 and 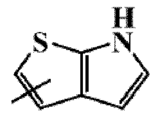

etc.

The term "alkoxy" refers to -O-(alkyl) and -O- (non-substituted cycloalkyl), wherein the alkyl is as defined above. Non-limiting examples of alkoxy include: methoxy, ethoxy, propoxy, butoxy, cyclopropoxy, cyclobutoxy, cyclopentyloxy, cyclohexyloxy. The alkoxy may optionally be substituted or unsubstituted. When it is substituted, the substituent is preferably one or more of groups independently selected from alkyl, alkenyl, alkynyl, alkoxy, alkylthio, alkylamino, halogen, mercapto, hydroxyl, nitro, cyano, cycloalkyl, heterocycloalkyl, aryl, heteroaryl, cycloalkoxy, heterocycloalkoxy, cycloalkylthio, heterocycloalkylthio, carboxyl or carboxylate.

The term "haloalkyl" refers to alkyl substituted with one or more halogens, wherein the alkyl is as defined above.

The term "haloalkoxy" refers to alkoxy substituted with one or more halogens, wherein the alkoxy is as defined above.

The term "hydroxyalkyl" refers to alkyl substituted with hydroxyl, wherein the alkyl is as defined above.

The term "alkylthio" refers to -S-(alkyl) and -S-(non-substituted cycloalkyl), wherein the alkyl is as defined above. Non-limiting examples of alkylthio include: methylthio, ethylthio, propylthio, butylthio, cyclopropylthio, cyclobutylthio,

cyclopentylthio, and cyclohexylthio. The alkylthio may be optionally substituted or unsubstituted, and when it is substituted, the substituent is preferably one or more of groups independently selected from alkyl, alkenyl, alkynyl, alkoxy, alkylthio, alkylamino, halogen, mercapto, hydroxyl, nitro, cyano, cycloalkyl, heterocycloalkyl, aryl, heteroaryl, cycloalkoxy, heterocycloalkoxy, cycloalkylthio, heterocycloalkylthio, carboxyl or carboxylate.

"Alkenyl" refers to alkenyl, as known as alkylene group, a straight chain or branched chain group comprising 2 to 20 carbon atoms, preferably alkenyl comprising 2 to 8 carbon atoms, more preferably alkenyl comprising 2 to 6 carbon atoms, and most preferably alkenyl comprising 2 to 4 carbon atoms. The alkenyl can be further substituted with other related groups, such as: alkyl, alkenyl, alkynyl, alkoxy, alkylthio, alkylamino, halogen, mercapto, hydroxyl, nitro, cyano, cycloalkyl, heterocycloalkyl, aryl, heteroaryl, cycloalkoxy, heterocycloalkoxy, cycloalkylthio, heterocycloalkylthio, carboxyl or carboxylate.

"Alkynyl" refers to a straight chain or branched chain group comprising 2 to 20 carbon atoms, preferably alkynyl comprising 2 to 8 carbon atoms, more preferably alkynyl comprising 2 to 6 carbon atoms, and most preferably alkynyl comprising 2 to 4 carbon atoms. The alkynyl can be further substituted with other related groups, such as: alkyl, alkenyl, alkynyl, alkoxy, alkylthio, alkylamino, halogen, mercapto, hydroxyl, nitro, cyano, cycloalkyl, heterocycloalkyl, aryl, heteroaryl, cycloalkoxy, heterocycloalkoxy, cycloalkylthio, heterocycloalkylthio, carboxyl or carboxylate.

"Hydroxyl" refers to the -OH group.

"Halogen" refers to fluorine, chlorine, bromine or iodine.

"Amino" refers to $-NH_2$.

"Cyano" refers to -CN.

"X is selected from A, B, or C", "X is selected from A, B, and C", "X is A, B, or C", and "X is A, B, and C" are different terms expressing the same meaning, that is, X can be any one or more of A, B, and C.

The hydrogen atoms described in the present invention can be replaced by its isotope deuterium, and any hydrogen atom in the example compounds involved in the present invention can also be replaced by a deuterium atom.

"Optional" or "optionally" means that the event or circumstance described subsequently may but need not occur, and the description includes the situations in which the event or circumstance occurs or does not occur. For example, "heterocyclic groups optionally substituted with alkyl" means that alkyl may but do not need to be present, and the description includes cases in which heterocyclic groups are substituted with alkyl and cases in which heterocyclic groups are not substituted with alkyl.

"Substituted" means that one or more hydrogen atoms in the group, preferably up to 5, more preferably 1 to 3 hydrogen atoms, are independently substituted with a corresponding number of substituents. It goes without saying that the substituents are only in their possible chemical positions, and those skilled in the art can determine (either experimentally or theoretically) possible or impossible substitutions without undue effort. For example, amino or hydroxyl with a free hydrogen may be unstable when bound to a carbon atom with an unsaturated (such as olefinic) bond. Optional substituents include one or more of deuterium, halogen, amino, hydroxyl, cyano, oxo, thio, alkyl, alkenyl, alkynyl, deuterated alkyl, haloalkyl, hydroxyalkyl, alkoxy, alkylthio, haloalkoxy, cycloalkyl, heterocyclyl, aryl and heteroaryl, preferably deuterium, halogen, amino, hydroxyl, cyano, oxo, thio, $C_{1-6}$ alkyl, $C_{2-6}$ alkenyl, $C_{2-6}$ alkynyl, $C_{1-6}$ deuterated alkyl, $C_{1-6}$ haloalkyl, $C_{1-6}$ hydroxyalkyl, $C_{1-6}$ alkoxy, $C_{1-6}$ alkylthio, $C_{1-6}$ haloalkoxy, $C_{3-12}$ cycloalkyl, 3-12-membered heterocyclyl, $C_{6-14}$ aryl and 5-14-membered heteroaryl.

"Pharmaceutical composition" means a mixture of one or more of the compounds described herein or physiologically/pharmaceutically acceptable salts or prodrugs thereof with other chemical components, and other components such as physiologically/pharmaceutically acceptable carriers and excipients. The purpose of pharmaceutical compositions is to facilitate administration to living organisms and facilitate the absorption of active ingredients to exert biological activity.

"Pharmaceutically acceptable salts" refer to salts of the compounds of the present invention that are safe and effective when used in mammals and that have desirable biological activity.

## DETAILED DESCRIPTION OF THE INVENTION

The present invention will be further described below with reference to examples, but these Examples do not limit the scope of the present invention.

Example 1 (3R)-N-(4-(chlorodifluoromethoxy)phenyl)-2-(difluoromethyl)-3-methyl-3,4,5a,6-tetrahydro-5-oxa-1,2a,6,8-tetraazabenzo[4,5]cyclooctyl[1,2,3-cd]indene-11-carboxamide p-toluenesulfonate salt

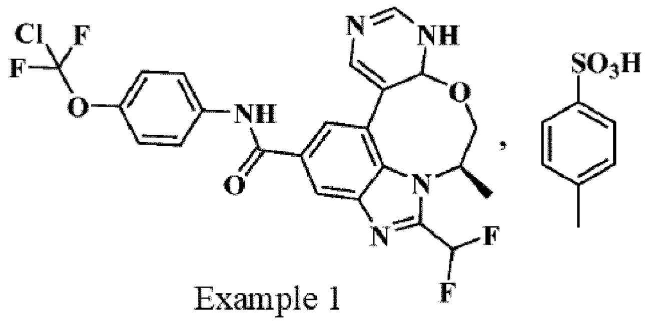

Example 1

Method 1:

Step 1: Synthesis of methyl 7-bromo-2-(difluoromethyl)-1H-difluoromethyl-benzo[d]imidazol-5-carboxylate

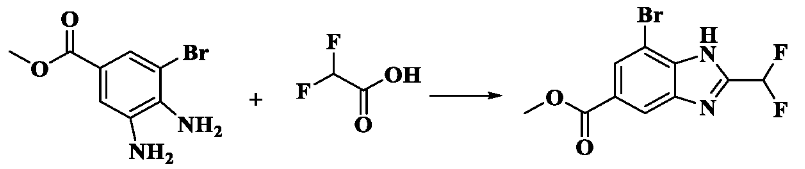

1-1 1-2 1-3

To a 100 mL reaction flask, 20 g of methyl 3,4-diamino-5-bromobenzoate and 60 mL of difluoroacetic acid were added, stirred for 10 to 30 min, and subjected to nitrogen replacement for three times. The temperature was heated to 70°C, and the reaction was stirred for 18 to 20 h. After the reaction was complete, 100 mL of ethyl acetate was added, the organic phase was adjusted to pH=7 with an aqueous solution of sodium bicarbonate, and the mixture was stirred for 5 to 10 min. The organic phase was collected, and the aqueous phase was discarded. The organic phase was washed with 50 mL of an added sodium chloride solution and dried over anhydrous sodium sulfate. The system was filtered, concentrated to dryness, and subjected to column chromatography to obtain 22.3 g of a product, with a yield of 90%.

Step 2: Synthesis of methyl (R)-7-bromo-2-(difluoromethyl)-1-[1-hydroxyprop-2-yl]-1H-benzo[d]imidazol-5-carboxylate

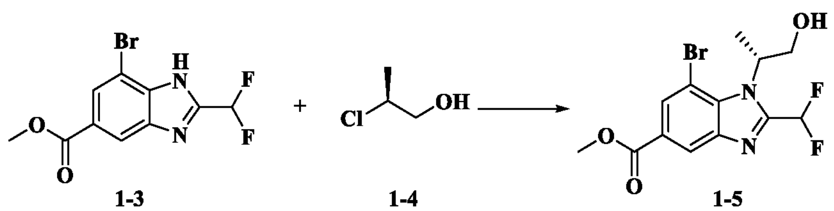

1-3 1-4 1-5

To a 500 mL reaction flask, 20 g of methyl 7-bromo-2-(difluoromethyl)-1H-difluoromethyl-benzo[d]imidazol-5-carboxylate, 4.8 g of cetyl trimethyl ammonium bromide, 18.1 g of potassium carbonate and 200 mL of acetonitrile were added, and a 9.3 g/50 mL (S)-2-chloro-1-propanol/acetonitrile solution was added dropwise with stirring at room temperature. After the dropwise addition was completed, the mixture was heated to reflux to react for 8 h. After the reaction was completed, the reaction mixture was cooled to room temperature, poured into ice water to precipitate a solid, and purified by column chromatography to obtain 16.3 g of a product, with a yield of 60%.

Step 3: Synthesis of methyl (R)-2-(difluoromethyl)-1-[1-hydroxyprop-2-yl]-7-(pyrimidin-5-yl)-1H-benzo[d]imidazol-5-carboxylate

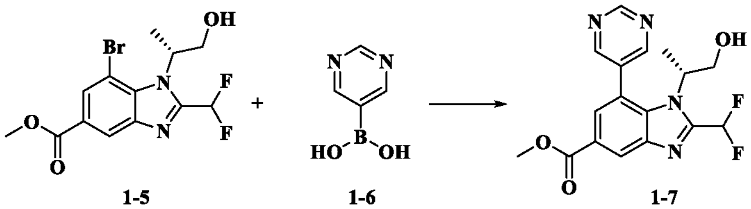

1-5 1-6 1-7

To a 250 mL three-necked flask, 15 g of methyl (R)-7-bromo-2-(difluoromethyl)-1-[1-hydroxyprop-2-yl]-1H-benzo[d]imidazol-5-carboxylate, 16.2 g of potassium acetate, 7.9 g of potassium fluoride, 105 mL of 1,4-dioxane, and 15 mL of water were added, and the system was subjected to nitrogen replacement for 3 times. 2.4 g of $Pd(PPh_3)_4$ was added, nitrogen replacement was carried out for 3 times, the system was heated to 80°C, and a solution of 5-pyrimidineboronic acid in tetrahydrofuran (6.1g/15mL) was added dropwise. After the dropwise addition was completed, the reaction was further stirred for 5 h, and after TLC monitoring showed that the raw materials reacted completely, the system was cooled to room temperature. 250 mL of water, 120 mL of ethyl acetate, and 3 g of N-acetyl cysteine were added to the reaction system, the system was stirred for 0.5 h, and 3 L of a saturated sodium bicarbonate solution was added. The system was separated into layers, and the aqueous phase was extracted with 120 mL of ethyl acetate. The organic phases were combined, washed with 5 L of a saturated sodium chloride solution, concentrated to dryness, and purified by column chromatography to obtain 10.3 g of a product with a yield of 69%.

Step 4: Synthesis of methyl (3R)-2-(difluoromethyl)-3-methyl-3,4,5a,6-tetrahydro-5-oxa-1,2a,6,8-tetraazabenzo[4,5]cyclooctyl[1,2,3-cd]indene-11-carboxylate

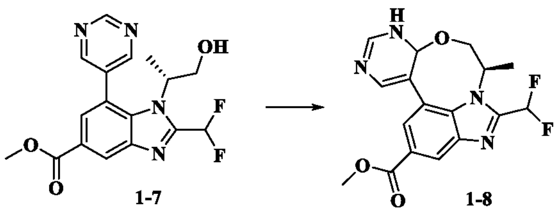

1-7 1-8

To a 250 mL reaction flask, 10 g of methyl (R)-2-(difluoromethyl)-1-[1-hydroxypropyl-2-yl]-7-(pyrimidin-5-yl)-1H-benzo[d]imidazol-5-carboxylate, 2.6 g of methanesulfonic acid and 150 mL of methylene chloride were added, stirred at room temperature for 1 h, filtered and dried to obtain 11.5 g of a product, with a yield of 91%.

Step 5: Synthesis of (3R)-N-(4-(chlorodifluoromethoxy)phenyl)-2-(difluoromethyl)-3-methyl-3,4,5a,6-tetrahydro-5-oxa-1,2a,6,8-tetraazabenzo[4,5]cyclooctyl[1,2,3-cd]indene-11-carboxamide

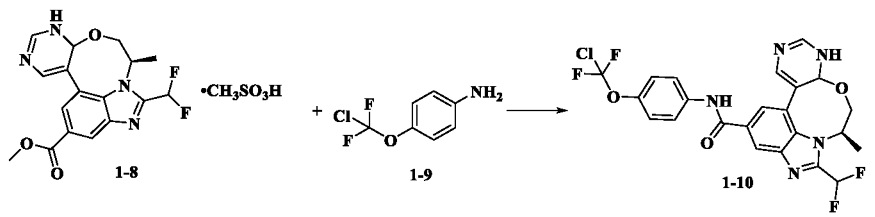

1-8 1-9 1-10

To a 250 mL reaction flask, 10 g of compound 1-8, 5.8 g of 4-(chlorodifluoromethoxy)aniline and 100 mL of tetrahydrofuran were added, and 44 mL of lithium bistrimethylsilylamide (1.0 M in THF) was added dropwise at 5-10°C. After the dropwise addition was completed, the reaction was stirred at room temperature for 15 h. 100 mL of a saturated aqueous ammonium chloride solution was added dropwise to the reaction mixture to quench the reaction, 200 mL of ethyl acetate was added for extraction, and the organic phase was concentrated to dryness. 100 mL of ethyl acetate was added to the concentrate, the concentrate was heated to 50°C, stirred for 0.5 h, and 50 mL of n-heptane was slowly added dropwise. The system was further stirred for 0.5 h, then cooled to 20-30°C, stirred for 3 h, filtered, and dried under vacuum to obtain 9.1 g of an off-white solid, with a yield of 82%.

Step 6: Synthesis of (3R)-N-(4-(chlorodifluoromethoxy)phenyl)-2-(difluoromethyl)-3-methyl-3,4,5a,6-tetrahydro-5-oxa-1,2a,6,8-tetraazabenzo[4,5]cyclooctyl[1,2,3-cd]indene-11-carboxamide 4-methylbenzenesulfonate salt

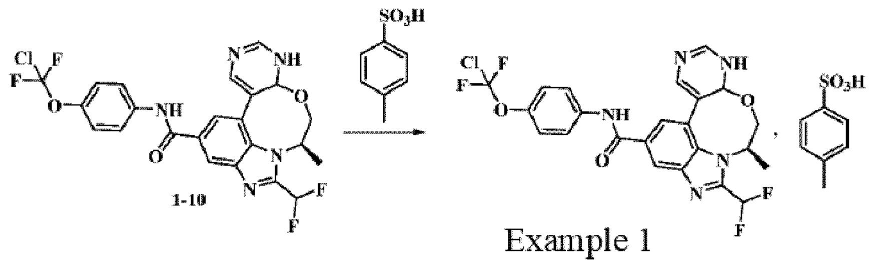

1-10

Example 1

9 g of compound 1-10 was weighed, 90 mL of acetonitrile was added, and the mixture was heated to 50°C, heated and stirred for 30 min. A solution of p-toluenesulfonic acid in ethanol (4g/20mL) was added, and then the system was incubated to react for 22-24 h. The mixture was filtered, and the filter cake was vacuum dried at 50°C for 24 h to obtain 10.1 g of a product with a yield of 84%.

$^1$HNMR(400Hz, DMSO-$d_6$) δ 10.51 (s, 1H), 8.68 (s, 1H), 8.35 (s, 1H), 8.58-8.59 (d, *J* = 1.6 Hz, 1H), 7.92-7.95 (d, *J* = 9.2 Hz, 2H), 7.84-7.85 (d, *J* = 1.6 Hz, 1H), 7.58-7.62 (m, 1H), 7.56 (t, *J* = 52.0 Hz, 1H), 7.48-7.50 (d, *J* = 7.6 Hz, 2H), 7.36-7.38 (d, *J* = 9.2 Hz, 2H), 7.11-7.13 (d, *J* = 7.6 Hz, 2H), 6.93 (br, 1H), 5.05 (s, 1H), 4.34-4.39 (m, 1H), 3.47-3.60 (m, 2H), 2.29 (s, 3H), 1.36-1.38 (d, *J* = 7.2 Hz, 3H).

MS (ESI, m/z): 524.2 [M-$C_7H_8O_3S$+H]$^+$.

Method 2:

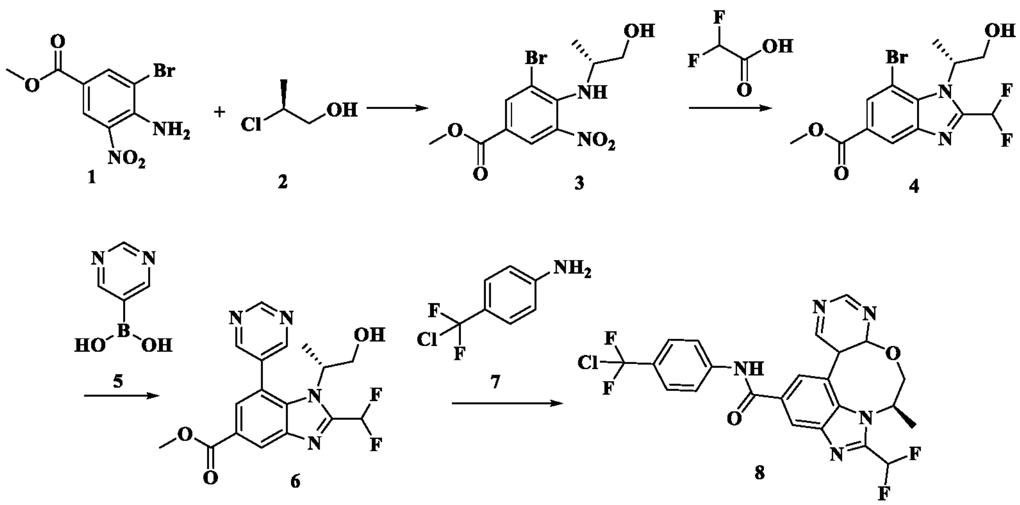

1 2 3 4 5 6 7 8

To a 500 mL reaction flask, 20 g of methyl 4-amino-3-bromo-5-nitrobenzoate (compound 1), 5.30 g of cetyl trimethyl ammonium bromide, 20.07 g of potassium carbonate and 200 mL of acetonitrile were added. A 10.3 g/ 50mL (*S*)-2-chloro-1-propanol/acetonitrile solution was added dropwise with stirring at room temperature. After the dropwise addition was completed, the mixture was heated to reflux to react for 8 h. After the reaction was completed, the reaction mixture was concentrated to dryness, ethyl acetate and water were added to the residue, and the mixture was extracted. The organic phase was washed with saturated sodium chloride and dried over anhydrous sodium sulfate. The system was filtered, and the filtrate was purified by column chromatography to obtain 13.32 g of compound 3, with a yield of 55%.

To a 100 mL reaction flask, 10 g of methyl (R)-3-bromo-4-[(1-hydroxyisopropan-2-yl)amino]-5-nitrobenzoate (compound 3) and 38 mL of difluoroacetic acid were added, 16.76 g of iron powder was added at room temperature, and the mixture was heated to 40-50°C to react for 3 h. The reaction system was cooled, and ethyl acetate was added thereto. The reaction system was filtered. The filtrate was washed once with water and saturated sodium chloride respectively, dried over anhydrous sodium sulfate, and filtered. The filtrate was concentrated and directly put into the

next reaction step.

To a 250 mL three-necked flask, 15 g of methyl (R)-7-bromo-2-(difluoromethyl)-1-[1-hydroxyprop-2-yl]-1H-benzo[d]imidazol-5-carboxylate (compound 4), 16.2 g of potassium acetate, 7.9 g of potassium fluoride, 105 mL of 1,4-dioxane, and 15 mL of water were added, and the system was subjected to nitrogen replacement for 3 times. 2.4 g of $Pd(PPh_3)_4$ was added, nitrogen replacement was carried out for 3 times, the system was heated to 80°C, and a solution of 5-pyrimidineboronic acid in tetrahydrofuran (6.1g/15mL) was added dropwise. After the dropwise addition was completed, the reaction was further stirred for 5 h, and after TLC monitoring showed that the raw materials reacted completely, the system was cooled to room temperature. 250 mL of water, 120 mL of ethyl acetate, and 3 g of N-acetyl cysteine were added to the reaction system, the system was stirred for 0.5 h, and 3 L of a saturated sodium bicarbonate solution was added. The system was separated into layers, and the aqueous phase was extracted with 120 mL of ethyl acetate. The organic phases were combined, washed with 5 L of a saturated sodium chloride solution, concentrated to dryness, and purified by column chromatography to obtain 10.3 g of compound 6 with a yield of 69%.

To a 250 mL reaction flask, 10 g of compound 6, 5.8 g of 4-(chlorodifluoromethoxy)aniline and 100 mL of tetrahydrofuran were added, and 44 mL of lithium bistrimethylsilylamide (1.0 M in THF) was added dropwise at 5-10°C. After the dropwise addition was completed, the system was stirred to react at room temperature for 15 h. 100 mL of a saturated aqueous ammonium chloride solution was added dropwise to the reaction mixture to quench the reaction, 200 mL of ethyl acetate was added for extraction, and the organic phase was concentrated to dryness. 100 mL of ethyl acetate was added to the concentrate, the concentrate was heated to 50°C and stirred for 0.5 h, and 50 mL of n-heptane was slowly added dropwise. The system was further stirred for 0.5 h, then cooled to 20-30°C, stirred for 3 h, filtered, and dried under vacuum to obtain 9.1g of an off-white solid, with a yield of 82%.

$^1$HNMR(400Hz, Acetone-$d_6$) $\delta$ 10.13 (s, 1H), 9.51 (s, 1H), 9.37 (s, 1H), 9.09-9.10 (d, $J$ = 2.4 Hz, 1H), 7.90-7.92 (m, 3H), 7.7.81 (s, 1H), 7.74 (t, $J$ = 52.4 Hz, 1H), 7.27-7.29 (d, $J$ = 8.4 Hz, 2H), 6.04 (s, 1H), 4.66-4.67 (m, 1H), 3.98-4.04 (m, 1H), 3.73-3.77 (m, 1H), 1.56-1.58 (d, $J$ = 6.8 Hz, 3H).

For the preparation of other examples, refer to Example 1.

| Example No. | Structure | ESI-MS $[M-C_7H_8O_3S+H]^+$ |
|---|---|---|
| 2 | 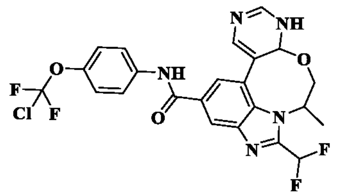 | 524.1 |
| 3 | 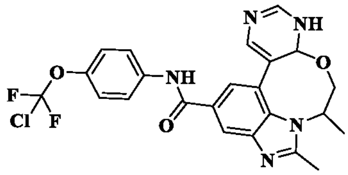 | 488.2 |
| 4 | 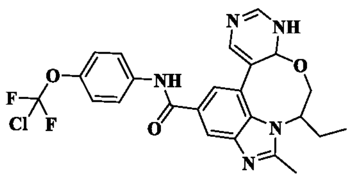 | 502.3 |
| 5 | 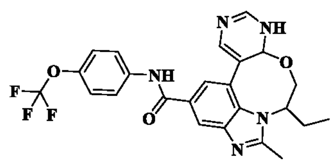 | 486.2 |

(continued)

| Example No. | Structure | ESI-MS $[M-C_7H_8O_3S+H]^+$ |
|---|---|---|
| 6 | 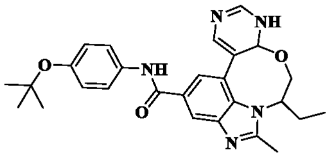 | 474.3 |
| 7 | 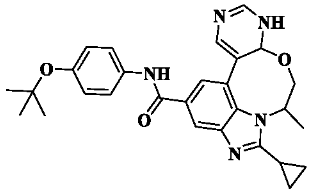 | 486.3 |
| 8 | 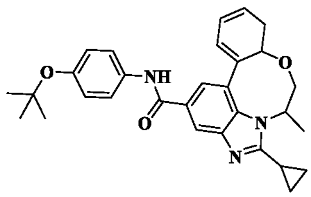 | 484.2 |
| 9 | 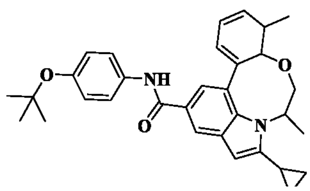 | 497.3 |

## Biological test evaluation

The present invention will be further described and explained below with reference to test examples, but these examples are not intended to limit the scope of the present invention.

### I. Study on the inhibitory activity of the compounds of the present invention against ABL1 WT kinase

1. Test purpose

To evaluate the in vitro inhibitory activity of the compounds of the present invention against ABL1 WT kinase.

2. Test methods

Capillary electrophoresis was used to detect the phosphorylation conversion rate of the substrate peptide to determine the $IC_{50}$ value of the test compound for inhibiting the kinase (ABL1-WT). The maximum concentration of the compound tested in the test was 1000 nM, with 3-fold dilution, and a total of 12 concentrations (1000 - 0.0056 nM). First, an enzyme reaction system (the concentration of enzyme ABL1-WT was 1.3 nM, the concentration of substrate FLPeptide2 was 1.5 $\mu$M, and the reaction factor was 10 mM $MgCl_2$) was prepared. After incubation at room temperature for 30 min, 5 $\mu$L of 4×ATP solution was added to start the enzyme reaction. After reacting at room temperature for 90 min, the reaction was stopped by adding a stop buffer (containing 0.5 M EDTA). The sample was analyzed using an EZ reader (analysis conditions: pressure -1.5PSI, upper voltage current -2250V, lower voltage current -500V, separation time 40 sec, and system delay 100.0 sec).

3. Data processing

From the conversion rate read by EZ Reader, the remaining activity was calculated according to the following formula.

$$\text{Remaining Activity (\%)} = 100\% \times \frac{CR_{Sample} - CR_{LC}}{CR_{HC} - CR_{LC}}$$

$IC_{50}$ was calculated using XLfit, and $IC_{50}$ was calculated by Formula 201 as the fitting formula.

4. Test results

**Table 1. In vitro enzymatic inhibitory activity of the compounds of the present invention against ABL1 WT kinase**

| Compound | Maximum concentration (μM) | ABL1-WT $IC_{50}$ (nM) |
|---|---|---|
| Example 1 | 1 | 0.43 |

In vitro enzymatic tests show that the compounds of the present invention exhibit good inhibitory activity against ABL1 WT kinase.

**II. Study on the inhibitory activity against proliferation of BaF3 cell strain overexpressing different BCR-ABL1 fusion mutations**

1. Test purpose

To evaluate the inhibitory activity of the compound of the present invention against proliferation of the Ba/F3 BCR-ABL1-T315I, Ba/F3 BCR-ABL1-E255K, Ba/F3 BCR-ABL1-E255V, and Ba/F3 BCR-ABL1-G250E mutation models of mouse primary B cells cultured in vitro.

2. Cell strains

| Cell line | Cell type |
|---|---|
| Ba/F3 BCR-ABL1-T315I | Mouse primary B cells<br>Overexpressing BCR-ABL1 fusion gene, and amino acid at position 315 on ABL1 being mutated to I |
| Ba/F3 BCR-ABL1-E255V | Mouse primary B cells<br>Overexpressing BCR-ABL1 fusion gene, and amino acid at position 255 on ABL1 being mutated to V |
| Ba/F3 BCR-ABL1-E255K | Mouse primary B cells<br>Overexpressing BCR-ABL1 fusion gene, and amino acid at position 255 on ABL1 being mutated to K |
| Ba/F3 BCR-ABL1-G250E | Mouse primary B cells<br>Overexpressing BCR-ABL1 fusion gene, and amino acid at position 250 on ABL1 being mutated to E |

3. Test methods

CellTiter-Glo® Luminescent Cell Viability Assay was used to detect the inhibitory effect of drugs on tumor cell proliferation and growth. The logarithmic growth phase cells cultured overnight were directly taken, blown, mixed and counted, the cell density was adjusted according to different cell density requirements, and the cells were prepared into

a cell suspension and plated on a 96-well plate. Drugs at different concentrations were added, 3 replicate wells were set for each concentration, and corresponding vehicle controls were eset. The maximum test concentration in Ba/F3 BCR-ABL1-T315I and Ba/F3 BCR-ABL1-E255K cells was 10000 nM, with 3.16-fold gradient dilution and a total of 9 concentrations (10000-1.0058 nM). The maximum concentration of the compounds detected in other cells was 500 nM, with 3.16-fold gradient dilution, and a total of 9 concentrations (500-0.0503 nM). The cells with the compound added were cultured for another 72 h at 37°C and 5% $CO_2$. The culture plate and its contents were equilibrated to room temperature, the CellTiter-Glo® reagent was added, the contents were mixed on a shaker for 5 min to induce cell lysis, the culture plate was further incubated at room temperature in the dark for 20 min, and the luminescence was read with a microplate reader.

4. Test results

**Table 2. The inhibitory activity of the compounds of the present invention against proliferation of BCR-ABL1 mutant cells**

| Compound | $IC_{50}$ (nM) | | | |
|---|---|---|---|---|
| | Ba/F3 BCR-ABL1-T315I | Ba/F3 BCR-ABL1-E255V | Ba/F3 BCR-ABL1-E255K | Ba/F3 BCR-ABL1-G250E |
| Example 1 | 46.76 | 7.15 | 26.27 | 5.36 |

The compound of the present invention can significantly inhibit the proliferation of Ba/F3 BCR-ABL1-T315I, E255K, E255V, and G250E mutant cells.

**III. Study on the inhibitory activity against proliferation of tumor cell strains with BCR-ABL1 fusion mutations**

1. Test purpose

To evaluate the inhibitory activity of the compounds of the present invention against proliferation of human erythroleukemia cells K562, human peripheral blood basophilic leukemia cells Ku812, and human chronic myelogenous leukemia cells KCL22-s and KCL22-r cultured in vitro.

2. Cell strains

| Cell line | Cell type |
|---|---|
| K562 | Human erythroleukemia cells BCR-ABL1 gene fusion mutation |
| Ku812 | Human peripheral blood basophilic leukemia cells BCR-ABL1 gene fusion mutation |
| KCL22-s | Human chronic myelogenous leukemia cells, BCR-ABL1 gene fusion mutation, imatinib-sensitive strain |
| KCL22-r | Human chronic myelogenous leukemia cells, BCR-ABL1 gene fusion mutation, imatinib-resistant strain |

3. Test methods

CellTiter-Glo® Luminescent Cell Viability Assay was used to detect the inhibitory effect of drugs on tumor cell proliferation and growth. The logarithmic growth phase cells cultured overnight were directly taken, blown, mixed and counted, the cell density was adjusted according to different cell density requirements, and the cells were prepared into a cell suspension and plated on a 96-well plate. Drugs at different concentrations were added, three replicate wells were set for each concentration, and corresponding solvent controls were set. The maximum concentration of compound detected was 500 nM, with 3-fold gradient dilution, and a total of 9 concentrations (500-0.076 nM). The cells with the compound being added were cultured for another 72 h at 37°C and 5% $CO_2$. The culture plate and its contents were equilibrated to room temperature, the CellTiter-Glo® reagent was added, the contents were mixed on a shaker for 3 min to induce cell lysis, the culture plate was further incubated at room temperature in the dark for 10 min, and the chemiluminescence signal value was determined by a microplate reader (BioTek SynergyH1).

4. Test results

**Table 3 The inhibitory activity of the compounds of the present invention against proliferation of BCR-ABL1 gene fusion mutant cells**

| Compound | $IC_{50}$ (nM) | | | |
|---|---|---|---|---|
| | K562 | Ku812 | KCL22-s | KCL22-r |
| Example 1 | 13.00 | 4.13 | 2.99 | 14.64 |

Test results show that the compound of the present invention can significantly inhibit the proliferation of tumor cells with BCR-ABL1 fusion mutations.

**IV. Study on drug efficacy on human chronic myeloid leukemia cell line KCL22-s xenograft tumor model**

1 Experimental purpose:

To evaluate the efficacy of the test compound on the human chronic myeloid leukemia cell strain KCL22-s in the BALB/c nude mouse subcutaneous xenograft tumor model.

2 Experimental operations and data processing:

2.1 Animals

BALB/c nude mice, 8-10 weeks.

2.2 Preparation for cell culture and cell suspension

a, A strain of KCL22-s cells was taken out from the cell bank, and the cells were resuscitated with RPMI-1640 medium (RPMI-1640+10% FBS+1% P/S). The resuscitated cells were placed in a cell culture flask (the cell type, date, name of the person for culture and so on were marked on the flask wall) and then placed in a CO2 incubator (the incubator temperature was 37°C and the CO2 concentration was 5%) for incubation.
b, The cells were passaged. After passage, the cells continued to be cultured in the CO2 incubator. This process was repeated until the cell number met the in vivo drug efficacy requirements.
c, The cultured cells were collected and counted with a fully automatic cell counter. The cells were resuspended in PBS according to the counting results to make a cell suspension (at a density of 5×107/mL), which was placed in an ice box for later use.

2.3 Cell inoculation

a, The nude mice were labeled with disposable ear tags for mice and rats before inoculation
b, When inoculated, the cell suspension was mixed evenly, 0.1-1 mL of the cell suspension was drawn using a 1 mL syringe, air bubbles were removed, and then the syringe was placed on an ice pack for later use.
c, The nude mouse was secured with the left hand, the right side of the nude mouse's back near the right shoulder (inoculation site) was disinfected with 75% alcohol, and inoculation was started after 30 seconds.
d, The experimental nude mice were inoculated in sequence (inoculation of 0.1 mL of cell suspension for each mouse).

2.4 Tumor measurement, grouping and dosing for tumor-bearing mice

a, According to the tumor growth, the tumor was measured on the 15th day after inoculation and the tumor size was

calculated.

Tumor volume calculation: Tumor volume (mm3) = length (mm) × width (mm) × width (mm) / 2

b, According to the body weight and tumor size of tumor-bearing mice, random grouping method was used to group the mice.

c, According to the grouping results, it started to administer the test drug (administration route: oral administration; dose: 1.5, 3, and 7.5 mg/kg; dosing volume: 10 mL/kg; dosing frequency: 1-2 times/day; dosing cycle: 15 days; and solvent: 0.5% HPMC K4M).

d, After starting to administer the test drug, the tumor was measured and weighed twice a week.

e, The animals were euthanized after the experiment.

f, The data were processed using Excel and other software. Calculation of compound tumor inhibition rate TGI (%): In the case of absence of tumor regression, TGI (%) = [1-(average tumor volume at the end of administration in a treatment group - average tumor volume at the beginning of administration in the treatment group)/(average tumor volume at the end of treatment in the solvent control group - average tumor volume at the beginning of treatment in the solvent control group)] × 100%. In the case of presence of tumor regression, TGI (%) = [1-(average tumor volume at the end of administration in a certain treatment group - average tumor volume at the beginning of administration in the treatment group)/average tumor volume at the beginning of administration in the treatment group] × 100%.

3 Experimental results:

**Table 4. Evaluation of the anti-tumor efficacy of the compounds of the present invention for the KCL22-s xenograft tumor model**

| Grouping | Tumor volume ($mm^3$) [a] | T/C (%) | TGI (%) |
|---|---|---|---|
| Vehicle bid | 2,034±235 | - | - |
| Imatinib 75 mg/kg qd | 954±220 | 46.92 | 56.03 |
| Example 1 3 mg/kg qd | 81±19 | 3.98 | 127.26 |
| Example 1 1.5 mg/kg bid | 143±35 | 7.05 | 98.20 |
| Example 1 3 mg/kg bid | 14±5 | 0.69 | 187.08 |
| Example 1 7.5 mg/kg bid | 11±7 | 0.52 | 190.62 |

The compound of the present invention has significant tumor inhibitory effect on the human chronic myelogenous leukemia cell line KCL22-s xenograft tumor model.

## Claims

**1.** A compound represented by formula (I), stereoisomers thereof or pharmaceutically acceptable salts thereof:

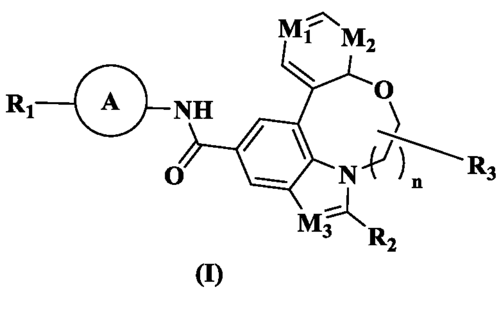

(I)

wherein:

ring A is selected from $C_{3-6}$ cycloalkyl, 4-7-membered heterocyclyl, $C_{6-10}$ aryl or 5-7-membered heteroaryl;
Ri is selected from hydrogen, deuterium, halogen, amino, hydroxyl, cyano, $C_{1-6}$ alkyl, $C_{2-6}$ alkenyl, $C_{2-6}$ alkynyl, $C_{1-6}$ deuterated alkyl, $C_{1-6}$ haloalkyl, $C_{1-6}$ hydroxyalkyl, $C_{1-6}$ alkoxy, $C_{1-6}$ alkylthio or $C_{1-6}$ haloalkoxy;
Mi is selected from N or $CR_a$;
$M_2$ is selected from $NR_a$ or $C(R_a)_2$;
$M_3$ is selected from N or $CR_a$;
$R_a$ is each independently selected from hydrogen, $C_{1-6}$ alkyl, $C_{1-6}$ deuterated alkyl, $C_{1-6}$ haloalkyl, $C_{1-6}$ hydroxyalkyl, $C_{1-6}$ alkoxy, $C_{1-6}$ alkylthio or $C_{1-6}$ haloalkoxy;
$R_2$ or $R_3$ are each independently selected from hydrogen, $C_{1-6}$ alkyl, $C_{1-6}$ deuterated alkyl, $C_{1-6}$ haloalkyl, $C_{1-6}$ hydroxyalkyl, $C_{1-6}$ alkoxy, $C_{1-6}$ alkylthio, $C_{1-6}$ haloalkoxy, $C_{3-6}$ cycloalkyl, 4-7-membered heterocyclyl, $C_{6-10}$ aryl or 5-7-membered heteroaryl; and
n is 0, 1 or 2.

2. The compound according to claim 1, stereoisomers thereof or pharmaceutically acceptable salts thereof, wherein,

ring A is $C_{6-10}$ aryl or 5-6-membered heteroaryl;
Ri is selected from $C_{1-3}$ alkyl, $C_{1-3}$ deuterated alkyl, $C_{1-3}$ haloalkyl, $C_{1-3}$ hydroxyalkyl, $C_{1-3}$ alkoxy, $C_{13}$ alkylthio or $C_{1-3}$ haloalkoxy;
Mi is selected from N or CH;
$M_2$ is selected from NH or $CH_2$;
$M_3$ is selected from N or CH; and
$R_2$ or $R_3$ are each independently selected from $C_{1-3}$ alkyl, $C_{1-3}$ deuterated alkyl, $C_{1-3}$ haloalkyl, $C_{1-3}$ hydroxyalkyl, $C_{1-3}$ alkoxy, $C_{1-3}$ alkylthio or $C_{1-3}$ haloalkoxy.

3. The compound according to claim 1 or 2, stereoisomers thereof or pharmaceutically acceptable salts thereof, wherein formula (I) is further as described in general formula (II) or (II-a):

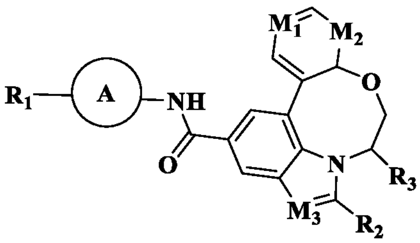

(II)

or

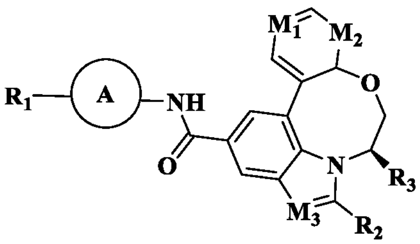

(II-a)

.

4. The compound according to claim 3, stereoisomers thereof or pharmaceutically acceptable salts thereof, wherein,

ring A is phenyl or pyridyl;
$R_1$ is selected from $C_{1-3}$ alkyl, $C_{1-3}$ haloalkyl, $C_{1-3}$ alkoxy or $C_{1-3}$ haloalkoxy;
$M_1$ is N;
$M_2$ is NH;
$M_3$ is N; and
$R_2$ or $R_3$ are each independently selected from hydrogen, $C_{1-3}$ alkyl or $C_{1-3}$ haloalkyl.

5. The compound according to any one of claims 1-4, stereoisomers thereof or pharmaceutically acceptable salts thereof, wherein,

$R_1$ is selected from $-CF_2$, $-CF_3$, $-CF_2Cl$, $-OCF_2$, $-OCF_3$ or $-OCF_2Cl$; and
$R_2$ or $R_3$ is each independently selected from $-CH_3$, $-CH_2CH_3$, $-CF_2$, $-CF_3$ or $-CF_2Cl$.

6. The compound according to claim 1, stereoisomers thereof or pharmaceutically acceptable salts thereof, wherein, the specific structure of the compound is as follows:

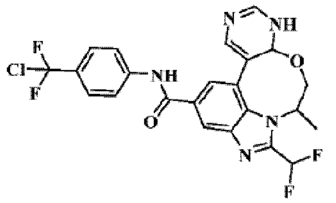 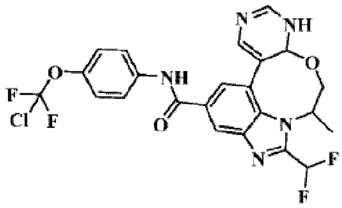 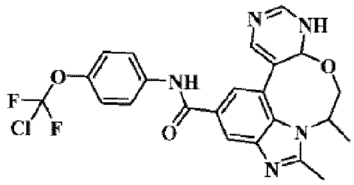

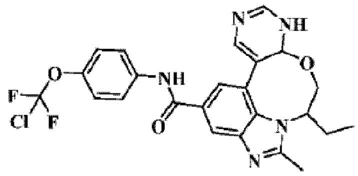 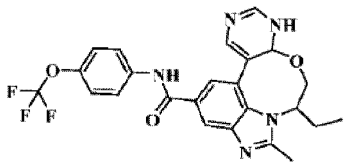 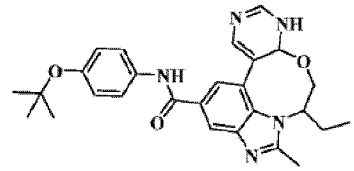

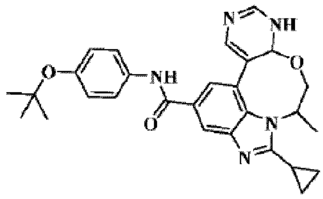 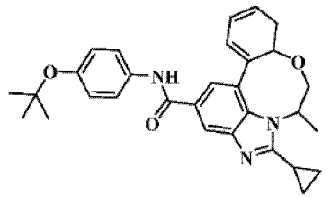 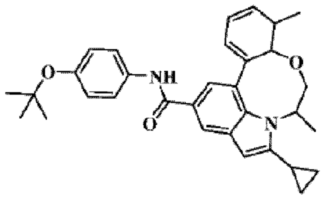

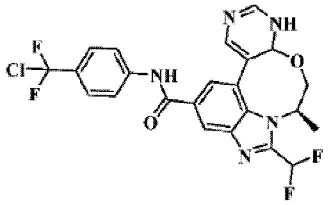 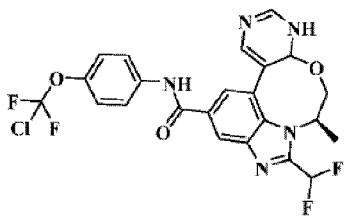 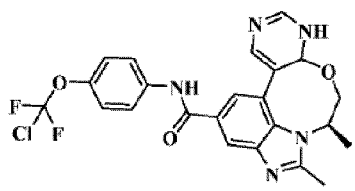

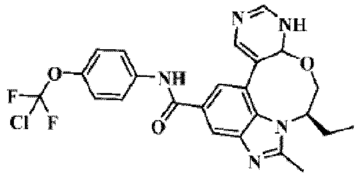 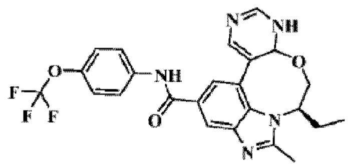 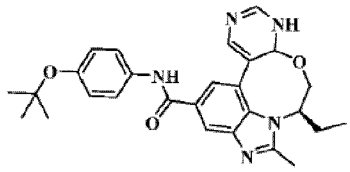

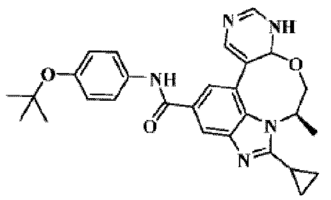 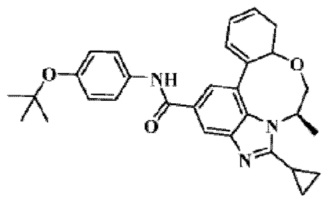 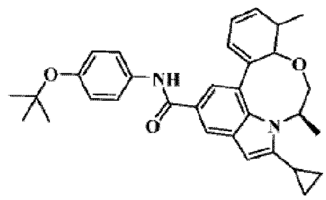.

7. The compound according to claims 1-6, stereoisomers thereof or pharmaceutically acceptable salts thereof, wherein the pharmaceutically acceptable salt is selected from sulfate, hydrochloride, ethyl sulfonate, methanesulfonate, p-toluenesulfonate, benzenesulfonate, isethionate or 1,5-naphthalenedisulfonate, preferably p-toluenesulfonate.

8. A pharmaceutical composition containing a therapeutically effective amount of the compound of any one of claims 1-7, stereoisomers thereof or pharmaceutically acceptable salts thereof, and one or more pharmaceutically acceptable carriers or excipients.

9. Use of the compound according to any one of claims 1-7, stereoisomers thereof or pharmaceutically acceptable salts thereof, and the pharmaceutical composition according to claim 8 in the preparation of ABL1 and/or BCR-ABL1 tyrosine kinase inhibitors.

**10.** Use of the compound according to any one of claims 1-7, stereoisomers thereof or pharmaceutically acceptable salts thereof, and the pharmaceutical composition according to claim 8 in the preparation of drugs for the treatment of leukemia-related diseases, wherein preferably, the leukemia is selected from chronic myelogenous leukemia, acute myeloid leukemia or acute lymphoblastic leukemia.

**11.** A method for preparing a compound of formula (II), stereoisomers thereof or pharmaceutically acceptable salts thereof,

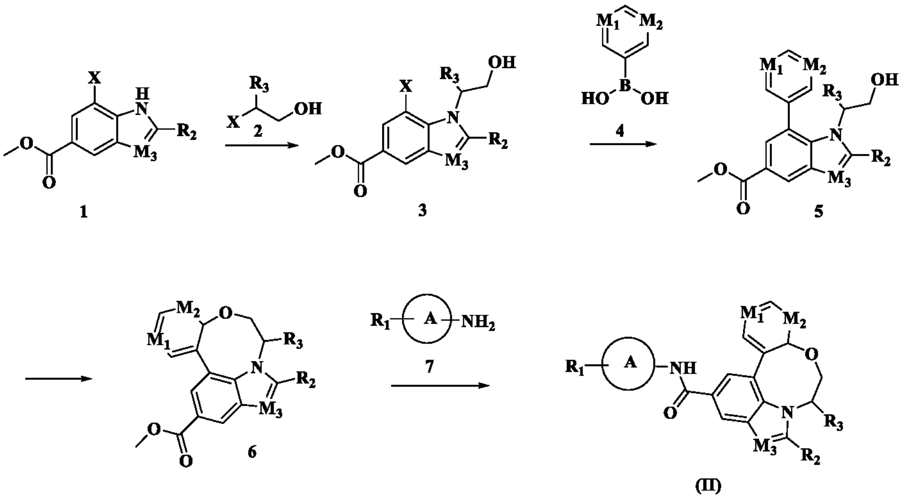

wherein compound 1 and compound 2 undergo substitution to prepare compound 3, compound 5 is prepared under the action of boric acid compound 4, and compound 5 is prepared into cyclic compound 6 under acidic conditions, which is subjected to condensation with compound 7 for preparation of compound (II), a stereoisomer thereof or a pharmaceutically acceptable salt thereof;

X is selected from halogen;
ring A, $R_1$-$R_3$ and $M_1$-$M_3$ are as defined in claim 3;
or,

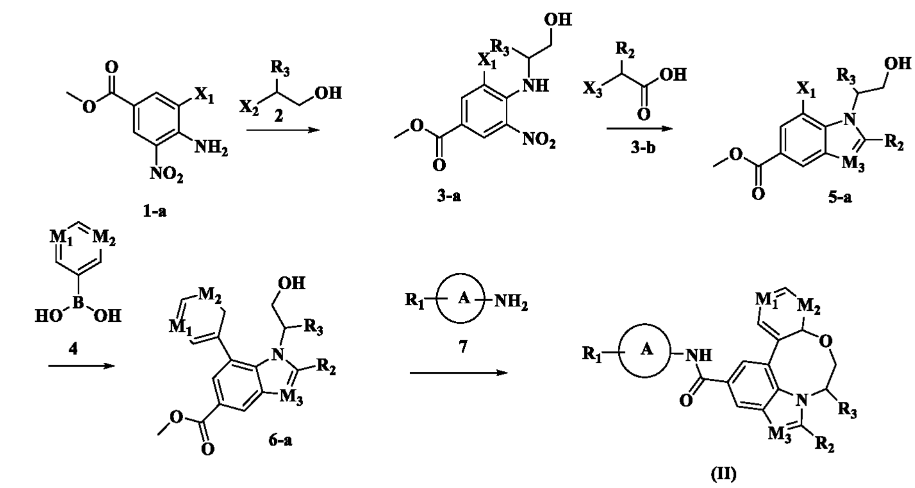

compound 1-a undergoes substitution with compound 2 to prepare compound 3-a, which reacts with 3-b to prepare compound 5-a, compound 6-a is prepared from compound 5-a under the action of boric acid compound 4, and compound 6-a and compound 7 undergo condensation to prepare compound (II);
optionally, compound (II) reacts with an acid to prepare a salt compound;
$X_1$, $X_2$ and $X_3$ are each independently selected from halogen;
the definitions of ring A, $R_1$-$R_3$ and $M_1$-$M_3$ are as defined in the general formula (II), preferably $M_3$ is N; and the acid is preferably p-toluenesulfonic acid.

**INTERNATIONAL SEARCH REPORT**

International application No.
**PCT/CN2022/122536**

**A. CLASSIFICATION OF SUBJECT MATTER**

C07D 267/22(2006.01)i; C07D 267/16(2006.01)i; C07D 498/22(2006.01)i; A61K 31/33(2006.01)i; A61K 31/55(2006.01)i; A61P 35/02(2006.01)i

According to International Patent Classification (IPC) or to both national classification and IPC

**B. FIELDS SEARCHED**

Minimum documentation searched (classification system followed by classification symbols)

C07D; A61K; A61P

Documentation searched other than minimum documentation to the extent that such documents are included in the fields searched

Electronic data base consulted during the international search (name of data base and, where practicable, search terms used)

CNTXT; WPABS; DWPI; CNKI; Registry(STN); CAPlus(STN); Marpat(STN); 豪森; 王霄; 酪氨酸; 蛋白; 激酶; 抑制; 白血病; 四氮杂; 环辛; 茚; HANSOH; tyrosine; kinase; inhibit+; abelson; ABL; protein; BCR-ABL; CML; AML; ALL; cyclooctane; indene; structural formula search

**C. DOCUMENTS CONSIDERED TO BE RELEVANT**

| Category* | Citation of document, with indication, where appropriate, of the relevant passages | Relevant to claim No. |
|---|---|---|
| A | US 2021101872 A1 (THE USA, AS REPRESENTED BY THE SECRETARY DEPT. OF HEALTH AND HUMAN SERVICES et al.) 08 April 2021 (2021-04-08) description, paragraphs [0066], [0111]-[0120], and [0160] | 1-11 |
| A | US 2004029850 A1 (ASTRAZENECA AB et al.) 12 February 2004 (2004-02-12) description, paragraphs [0005]-[0032] and [0104]-[0119] | 1-11 |
| A | CN 109790144 A (ASTAR BIOTECH LLC.) 21 May 2019 (2019-05-21) descriptions [0016] and [0020]-[0037], and embodiments 001-067 | 1-11 |
| A | CN 103113355 A (WUXI ALLNATURE BIOLOGICAL SCIENCE & TECHNOLOGY CO., LTD.) 22 May 2013 (2013-05-22) descriptions [0004]-[0017], and embodiments 1-7 | 1-11 |
| A | DERATT, L. G. et al. "Tandem Suzuki Coupling/Intramolecular Oxetane Ring Opening to Form Polycyclic Ring Systems" *Organic Letters*, Vol. 22, No. 15, 23 July 2020 (2020-07-23), pp. 5828-5832 | 1-11 |

☐ Further documents are listed in the continuation of Box C. ☑ See patent family annex.

* Special categories of cited documents:
- "A" document defining the general state of the art which is not considered to be of particular relevance
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **02 December 2022** | **15 December 2022** |

| Name and mailing address of the ISA/CN | Authorized officer |
|---|---|
| **China National Intellectual Property Administration (ISA/CN)**<br>**No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

**INTERNATIONAL SEARCH REPORT**
**Information on patent family members**

International application No.
**PCT/CN2022/122536**

| Patent document cited in search report | Publication date (day/month/year) | Patent family member(s) | Publication date (day/month/year) |
|---|---|---|---|
| US 2021101872 A1 | 08 April 2021 | WO 2019173761 A1 | 12 September 2019 |
| | | CL 2020002317 A1 | 08 January 2021 |
| US 2004029850 A1 | 12 February 2004 | SE 0003476 D0 | 28 September 2000 |
| | | JP 2004509954 A | 02 April 2004 |
| | | DE 60129829 D1 | 20 September 2007 |
| | | AT 369348 T | 15 August 2007 |
| | | WO 0226724 A1 | 04 April 2002 |
| | | US 2005192318 A1 | 01 September 2005 |
| | | AU 9048001 A | 08 April 2002 |
| | | EP 1324992 A1 | 09 July 2003 |
| | | ES 2288988 T3 | 01 February 2008 |
| CN 109790144 A | 21 May 2019 | JP 2019515932 A | 13 June 2019 |
| | | EP 3448852 A1 | 06 March 2019 |
| | | WO 2017186148 A1 | 02 November 2017 |
| CN 103113355 A | 22 May 2013 | None | |

Form PCT/ISA/210 (patent family annex) (January 2015)